# EUROPEAN PATENT SPECIFICATION

(11) **EP 2 170 270 B1**
(45) Date of publication and mention of the grant of the patent: **13.01.2016**
(21) Application number: 08756691.5
(22) Date of filing: 04.06.2008
(51) Int. Cl.: A61K 47/42, A61K 47/48, A61K 38/39, A61K 9/14, A61K 9/00, A61K 38/17

(54) **COMPOSITIONS FOR DELIVERING MEDICAMENTS INTO THE LUNGS, USES THEREOF**
ZUSAMMENSETZUNGEN ZUR ABGABE VON MEDIKAMENTEN IN DIE LUNGE UND IHRE VERWENDUNG
COMPOSITION À LIBÉRATION DES MÉDICAMENTS AUX POUMONS, EMPLOI DE CETTE COMPOSITION

(30) Priority: 05.06.2007 US 942026 P
(43) Date of publication of application: 07.04.2010
(73) Proprietor: PAKA PULMONARY PHARMACEUTICALS, INC., Acton MA 01720 (US)
(72) Inventor: GUARNIERI, Frank, Brooklyn, NY 11223 (US)
(74) Representative: Lock, Graham James
(86) International application number: PCT/US2008/065776
(87) International publication number: WO 2008/151235

(56) References cited:
- WO-A-03/011316
- WO-A-03/090682
- WO-A-2005/055994
- WO-A-2007/005672

## Description

### BACKGROUND

In pulmonary disorders including chronic obstructive pulmonary disease (COPD), chronic bronchitis, and emphysema, there is a chronic obstruction of air flow in and out of the lungs. The obstruction that manifests in these disorders is often permanent and progresses over time. Exacerbations, which are an acute worsening of respiratory function, result in increased morbidity and mortality.

Over the last few decades, research to treat chronic pulmonary disorders such as COPD has focused on identifying inhibitors of human neutrophil elastase (HNE). HNE is a protease capable of degrading numerous proteins including the structural proteins fibronectin, collagen, and elastin. When aberrantly expressed, HNE is one of the most destructive enzymes in the body. HNE is associated with tissue destruction and inflammation and is implicated in numerous pulmonary diseases including COPD, cystic fibrosis, and acute respiratory distress syndrome (ARDS) as well as other diseases of the body. However, the development of HNE inhibitors has been difficult and despite decades of research only one HNE treatment is currently on the market with approval for use only in Japan.

The development of HNE inhibitors and other drugs designed for lung treatment has focused on systemic treatments. A major obstacle with such an approach, whether the drug is delivered orally, parenterally, or by inhalation, is achieving meaningful residence times in the lungs. Thus, there remains an unmet need for effective lung treatments.

WO03/011316, WO2005055994, WO2007005672, WO03090682 disclose a pulmonary active agent (an elastase inhibitor or a corticosteroid or a bronchodilator or an antibiotic or a chemotherapeutic), but not in a covalent binding to a surface active agent.

### SUMMARY

The disclosure provides methods and compositions for delivering medicaments to the lungs according to claim 1. It is now appreciated that a key problem associated with treating lung diseases is the difficulty in obtaining sufficient residence times of active drug molecules in the lungs. The lungs are very adept at clearing foreign matter, such that active drug molecules may be cleared from the lung before the desired medicinal effect is achieved.

Pulmonary surfactants are secreted by Type II pneumocytes in the lungs to reduce surface tension within the alveoli therefore preventing alveolar collapse during expiration. Pulmonary surfactants, which are a complex of lipids and proteins, spread across the alveolar surface to lower surface tension and are maintained in the lung for extended periods. Therefore, the residence time of active drug molecules in the lung can be increased by covalently linking the active drug molecule to a surfactant lipid or protein. Administering active drug molecules covalently linked to a surfactant lipid or protein provides increased duration of action in the lung resulting in substantially fewer doses and better patient compliance, localization of the active drug molecule to the lung resulting in decreased in systemic toxicity, and significantly higher localized lung concentrations for enhanced efficacy.

In one aspect, the invention provides a drug composition, formulated for inhalation, comprising a surface active agent that has an affinity for the human alveolar/gas interface. The surface active agent comprises at least a portion of a mammalian lung surfactant polypeptide or mimic thereof that is substantially non-immunogeneic to humans.

The surface active agent is covalently bonded to a pulmonary active drug, which binds to an extracellular or cell-surface target accessible to the pulmonary/gas interface. The extracellular or cell-surface target may be, by way of example, elastase, TNF receptor, EGF receptor, adrenergic receptor, or P2X purinergic receptor. In certain embodiments, the surface active agent covalently bonded to a pulmonary active drug, which binds to an extracellular or cell surface target is administered to a subject suffering from lung disease, including, but not limited to tuberculosis, asthma, and lung cancer. For example, the agent responsible for tuberculosis (TB) avoids destruction and effects intracellular multiplication by down-regulating apoptosis in macrophages. Macrophages carrying TB overexpress the P2X purinergic receptor (Placido et al., Cell Immunol. 244:10-8 (2006)). P2X agonists, such as ATP, will induce apoptosis in these macrophages and thus kill the parasitic TB (Pfeiffer et al., J. Leukoc. Biol. 75:1173 (2004)). Benzoyl derivatives of ATP are potent extracellular agonists of the P2X receptor and can be covalently linked to the surface active agent and delivered via inhalation as a long duration TB treatment. Agonists for the β2-adrenergic receptor are well-established bronchodilators for the treatment of asthma (Anderson, Clin. Rev. Allergy Immunol. 31:119-30

(2006). The β2-adrenergic receptor, however, is ubiquitously expressed and thus repeated dosing will likely have deleterious systemic side effects. Covalently linking a β2-adrenergic receptor agonist to a surface active peptide will essentially isolate the active agent to the lung, thus avoiding or diminishing the potential systemic toxicities. The epidermal growth factor receptor (EGFR) has been validated as an anticancer target (Carney, Expert Rev. Mol. Diagn. 7:309-19 (2007) with products such as herceptin on the market to treat breast cancer. Matrix metalloproteases have been shown to release endogeneous agonists of EGFR via a cell surface shedding mechanism (Horiuchi et al., Mol. Biol. Cell 18:176-188 (2007)). Inhibiting the activation of EGFR is a potential treatment for nonsmall cell lung cancer (Y.H. Ling et al., Molecular Pharmacology 72:248-58 (2007)). EGFR may be deactivated in the lungs by attaching a small molecule inhibitor(s) of MMPs to lung surface active peptides to be delivered by inhalation. These examples including emphysema, tuberculosis, asthma, and nonsmall cell lung cancer demonstrate the generality of covalently linking therapeutic molecules to agents that preferentially reside in the lung with inhalation delivery.

In another embodiment, the surface active agent is covalently bonded to a pulmonary active drug and a cell membrane permeable transport molecule that enters the lung cells. Agents that function within the cell include, but are not limited to retinoids, survivin inhibitors, and caspase promoters.

In another embodiment, the surface active agent comprises a human lung surfactant or a non-human mammalian lung surfactant or a fraction thereof. Exemplary non-human mammalian lung surfactants include bovine, porcine, or ovine lung surfactants or a fraction thereof. The agent may comprise or be derived from a mammalian lung surfactant harvested from the lungs of a human or non-human mammal.

In another embodiment, the surface active agent comprises at least a portion of a mammalian lung surfactant polypeptide, an allelic variant thereof, or a synthetic mimic thereof. The agent may comprise a natural surfactant polypeptide, such as SP-A, SP-B, SP-C, SP-D, portions thereof, or mixtures thereof. The agent may comprise a mixture of SP-A, SP-B, SP-C, SP-D or portions thereof. Exemplary peptides include at least about a 5, 10, 15, 20, 25, 30, 35, 40, 45, or 50 amino acid fragment of a natural surfactant polypeptide. The surface active agent may comprise at least a portion of SP-B. Exemplary SP-B polypeptides include at least about a 5, 10, 15, 20, 25, 30, 35, 40, 45, or 50 amino acid fragment of SP-B. An SP-B peptide may be an amino-terminal peptide or a carboxy-terminal peptide. An exemplary SP-B peptide may be a 25-amino acid amino terminal peptide.

In another embodiment, the surface active agent comprises a synthetically produced peptide. A peptidomimetic may comprise at least one deletion or amino acid substitution mutant of a mammalian lung surfactant polypeptide. A peptidomimetic may comprise at least one deletion or amino acid substitution mutant of a human lung surfactant polypeptide.

In another embodiment, the surface active agent may comprise a surfactant polypeptide that is recombinantly produced. A recombinant mammalian lung surfactant polypeptide, such as SP-A, SP-B, SP-C, SP-D, or a portion thereof may be produced by expressing the DNA coding for SP-A, SP-B, SP-C, SP-D, or a portion thereof in a prokaryotic or eukaryotic expression system. Recombinant surfactant polypeptides may be the same or differ from mammalian lung surfactant polypeptides. A recombinant polypeptide may comprise at least one deletion or amino acid substitution mutant of a mammalian, preferably a human lung surfactant polypeptide.

In another embodiment, the surface active agent comprises both a surfactant polypeptide and a lipid.

The surface active agent is covalently bonded to a pulmonary active drug. The pulmonary active drug may be covalently linked to a surfactant protein or lipid. The pulmonary active drug may be covalently bonded to an amino- or carboxy-terminal amino acid or an internal amino acid of a surfactant polypeptide. In certain embodiments, more than one pulmonary active drug is bound to a surface active agent. In other embodiments, a single pulmonary active drug is bound to a surface active agent and mixed with at least one other pulmonary active drug bound to a surface active agent.

In one embodiment, the a pulmonary active drug molecule is extended with an amino acid or mimetic linker, such as a glycine linker, to create an unnatural amino acid that can be used in automatic peptide synthesis. The extended molecule (i.e., the drug plus the amino acid linker) can then be attached to the surface active agent through an amino- or hydroxyl- group.

The pulmonary active drug binds to an extracellular or cell-surface bound target that is accessible to the pulmonary/gas interface. The pulmonary active drug may be an elastase inhibitor, corticosteroid, bronchodilator, antibiotic, or chemotherapeutic agent. In certain embodiments, more than one pulmonary active drug may be covalently bonded to a surface active agent and administered in combination. When more than one pulmonary active drug is covalently bonded to a surface active agent, the drug may be the same drug, a member of the same drug class, or a member of a different drug class.

The drug composition is delivered to the lungs of a human patient by an inhalation device. Exemplary inhalation devices include fixed dose inhalers, metered dose inhalers, and nebulizers.

In another aspect, the invention provides a method for treating a subject suffering from or at risk of a lung disease. The method comprises administering a conjugate comprising a pulmonary active drug covalently bonded to a surface active agent characterized by an affinity for the human alveolar/gas interface, wherein the surface active agent comprises at least a portion of a mammalian lung surfactant polypeptide or a mimic thereof that is substantially non-immunogenic to humans. The conjugate is administered to the subject by inhalation in an amount effective to induce a drug effect in the lungs.

The method of administration targets the pulmonary active drug to the lungs of a subject in need thereof. The method of administration reduces the systemic bioavailability of the drug relative to inhalation administration of an unconjugated drug. The method of administration increases the residence time of the drug in the lung relative to inhalation administration of an unconjugated drug.

In one embodiment, the administration of a pulmonary active drug-surface active agent conjugate reduces the dosing frequency relative to administration of an unconjugated drug. The administration step may be repeated once daily, every other day, every three days, every four days, every five days, or weekly. The administration step may be conducted using an inhaler, an aerosol, particulates with or without propellants, metered dosages, or a nebulizer.

In certain embodiments, the subject in need of treatment is suffering from lung inflammation or disease or is at risk of suffering from a lung disease. The subject in need of treatment may be suffering from emphysema, chronic bronchitis, chronic obstructive pulmonary disease (COPD), asthma, respiratory distress disorder (RDS), pneumonia, tuberculosis or other bacterial infection, cystic fibrosis, and/or lung cancer.

### BRIEF DESCRIPTION OF DRAWINGS

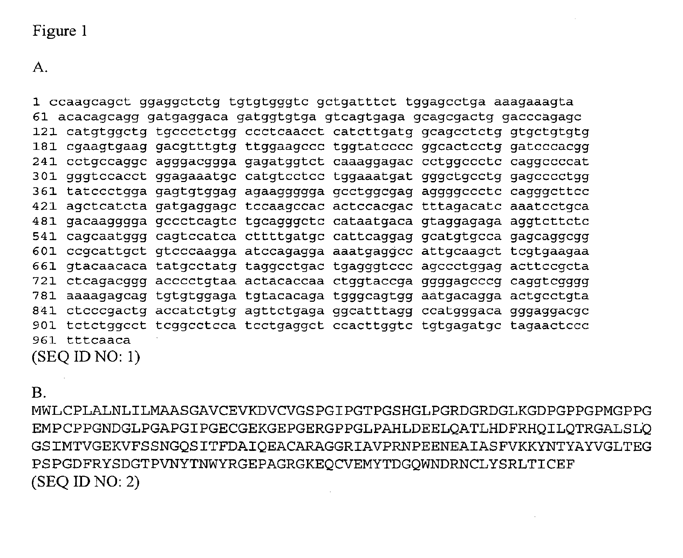

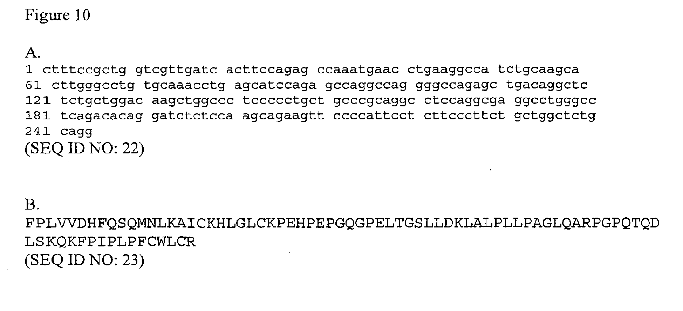

Figure 1A shows the nucleic acid sequence that encodes human surfactant protein A (SEQ ID NO: 1). Figure 1B shows the amino acid sequence for human surfactant protein A (SEQ ID NO: 2).

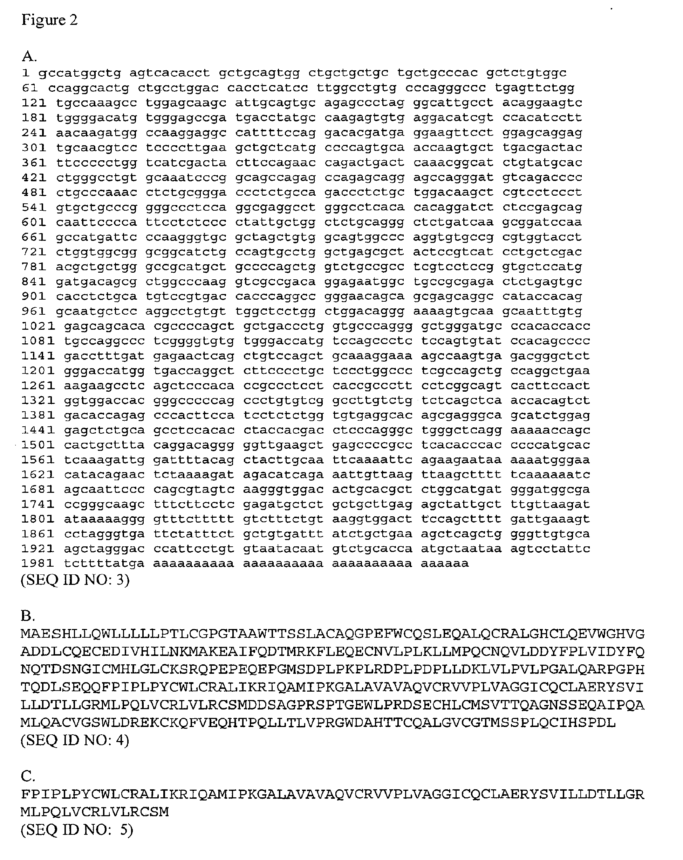

Figure 2A shows the nucleic acid sequence that encodes human surfactant protein B (SEQ ID NO: 3). Figure 2B shows the amino acid sequence for human surfactant protein B (SEQ ID NO: 4). Figure 2C shows the amino acid sequence for mature human surfactant protein B (SEQ ID NO: 5).

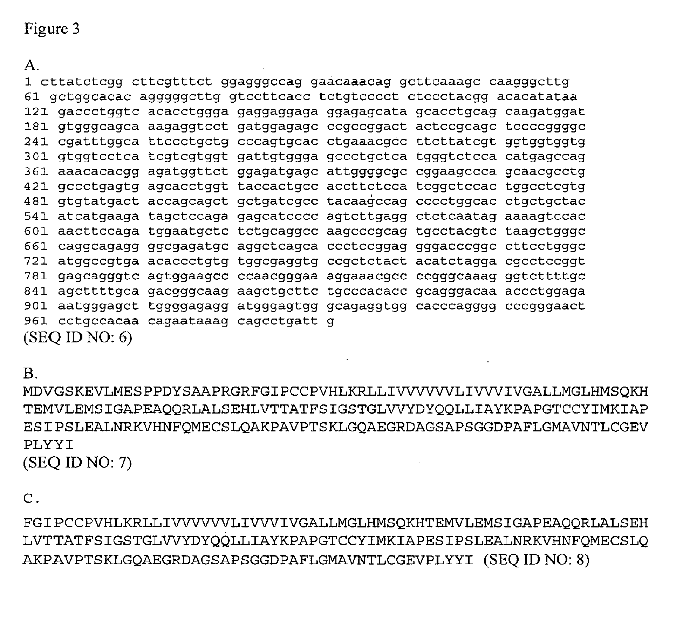

Figure 3A shows the nucleic acid sequence that encodes human surfactant protein C (SEQ ID NO: 6). Figure 3B shows the amino acid sequence for human surfactant protein C (SEQ ID NO: 7). Figure 3C shows the amino acid sequence for mature human surfactant protein C (SEQ ID NO: 8).

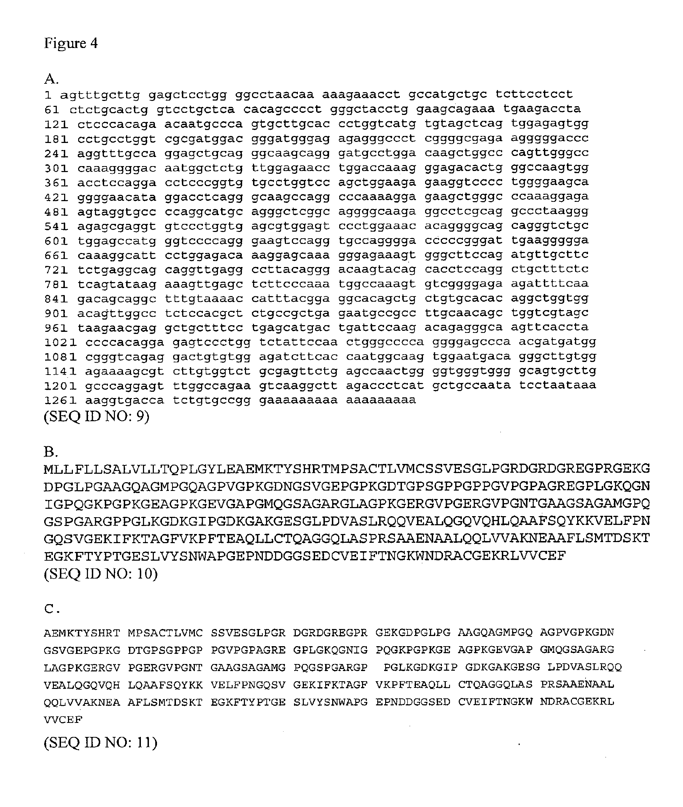

Figure 4A shows the nucleic acid sequence that encodes human surfactant protein D (SEQ ID NO: 9). Figure 4B shows the amino acid sequence for human surfactant protein D (SEQ ID NO: 10). Figure 4C shows the amino acid sequence for mature human surfactant protein D (SEQ ID NO: 11).

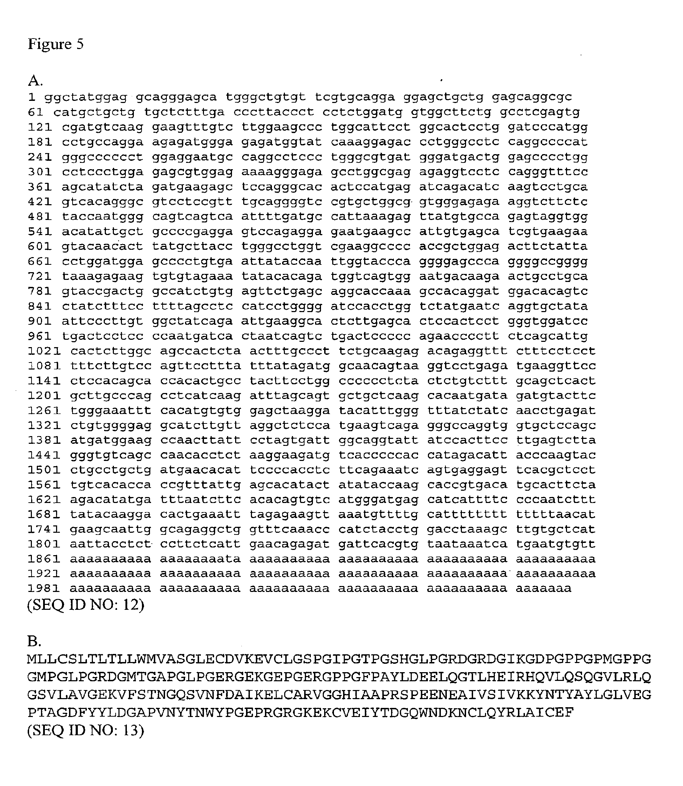

Figure 5A shows the nucleic acid sequence that encodes bovine surfactant protein A (SEQ ID NO: 12). Figure 5B shows the amino acid sequence for bovine surfactant protein A (SEQ ID NO: 13).

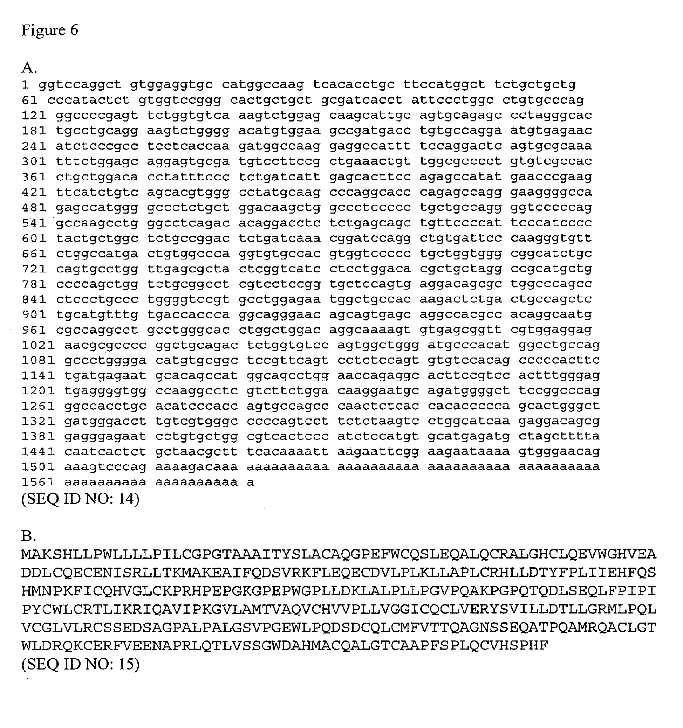

Figure 6A shows the nucleic acid sequence that encodes bovine surfactant protein B (SEQ ID NO: 14). Figure 6B shows the amino acid sequence for bovine surfactant protein B (SEQ ID NO: 15).

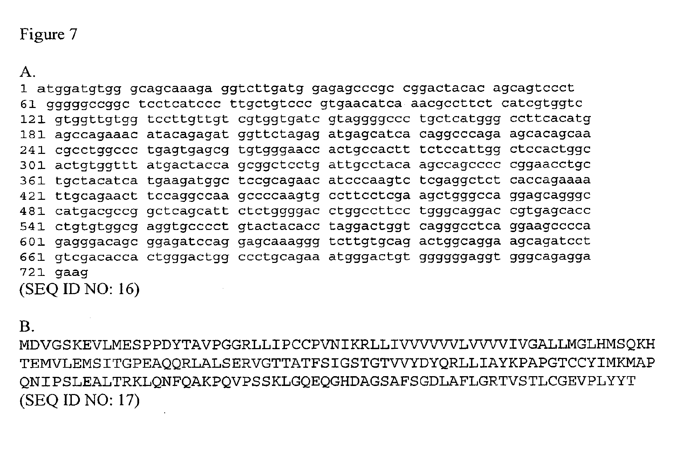

Figure 7A shows the nucleic acid sequence that encodes bovine surfactant protein C (SEQ ID NO: 16). Figure 7B shows the amino acid sequence for bovine surfactant protein C (SEQ ID NO: 17).

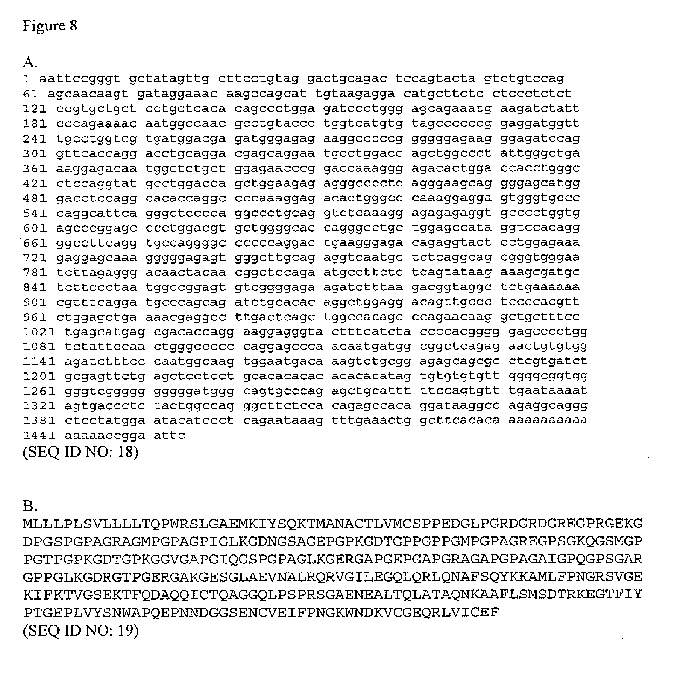

Figure 8A shows the nucleic acid sequence that encodes bovine surfactant protein D (SEQ ID NO: 18). Figure 8B shows the amino acid sequence for bovine surfactant protein D (SEQ ID NO: 19).

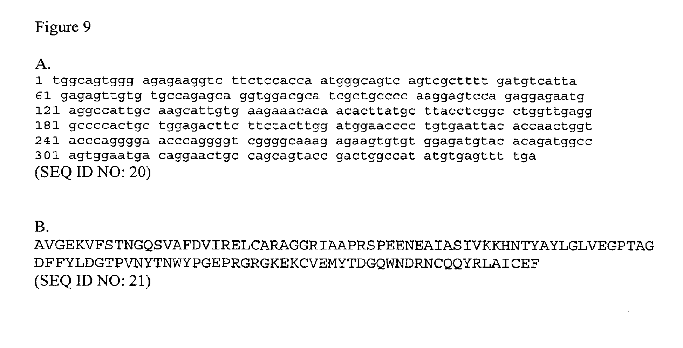

Figure 9A shows the nucleic acid sequence that encodes porcine surfactant protein A (SEQ ID NO: 20). Figure 9B shows the amino acid sequence for porcine surfactant protein A (SEQ ID NO: 21).
Figure shows the nucleic acid sequence that encodes a partial porcine surfactant protein B (SEQ ID NO: 22). Figure 10B shows a partial amino acid sequence for porcine surfactant protein B (SEQ ID NO: 23).

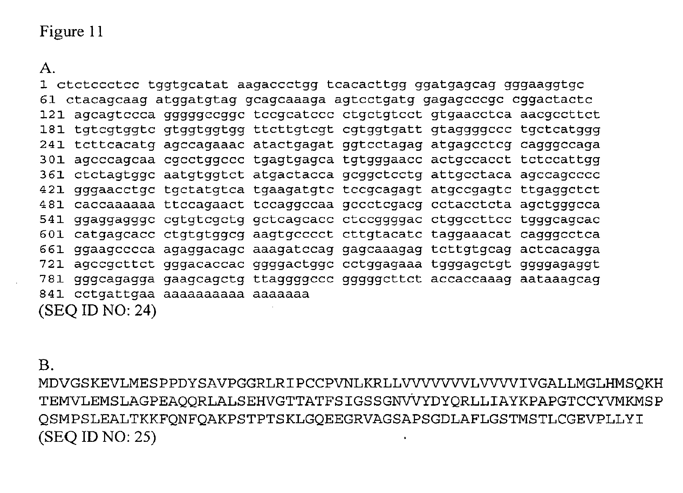

Figure 11A shows the nucleic acid sequence that encodes porcine surfactant protein C (SEQ ID NO: 24). Figure 11B shows the amino acid sequence for porcine surfactant protein C (SEQ ID NO: 25).

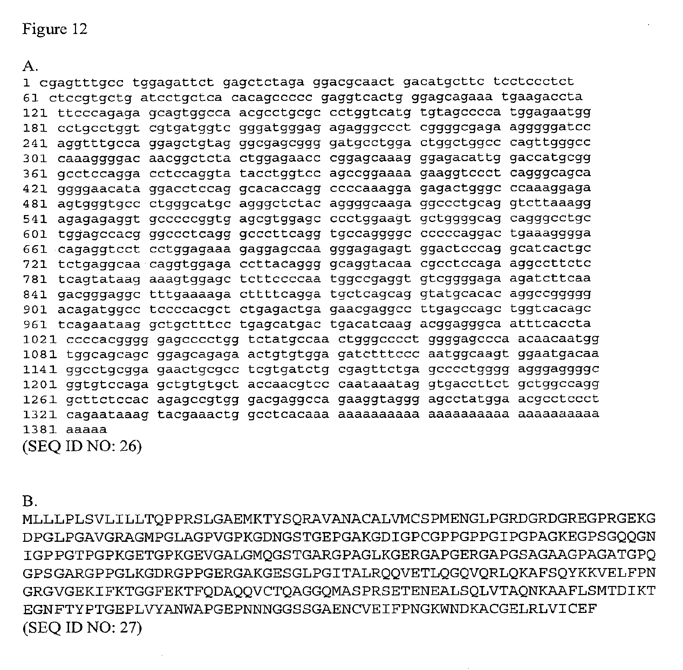

Figure 12A shows the nucleic acid sequence that encodes porcine surfactant protein D (SEQ ID NO: 26). Figure 12B shows the amino acid sequence for porcine surfactant protein D (SEQ ID NO: 27).

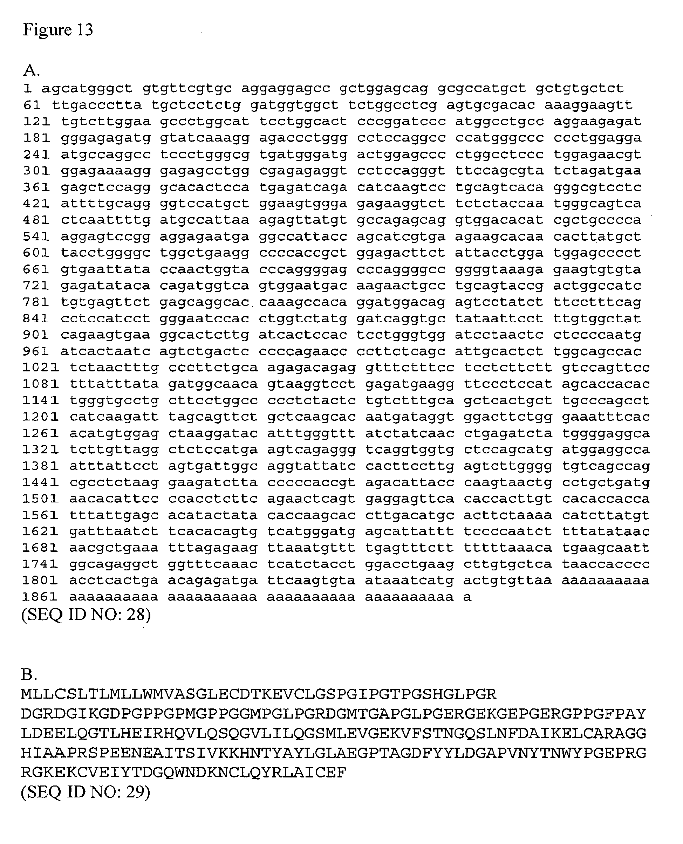

Figure 13A shows the nucleic acid sequence that encodes ovine surfactant protein A (SEQ ID NO: 28). Figure 13B shows the amino acid sequence for ovine surfactant protein A (SEQ ID NO: 29).

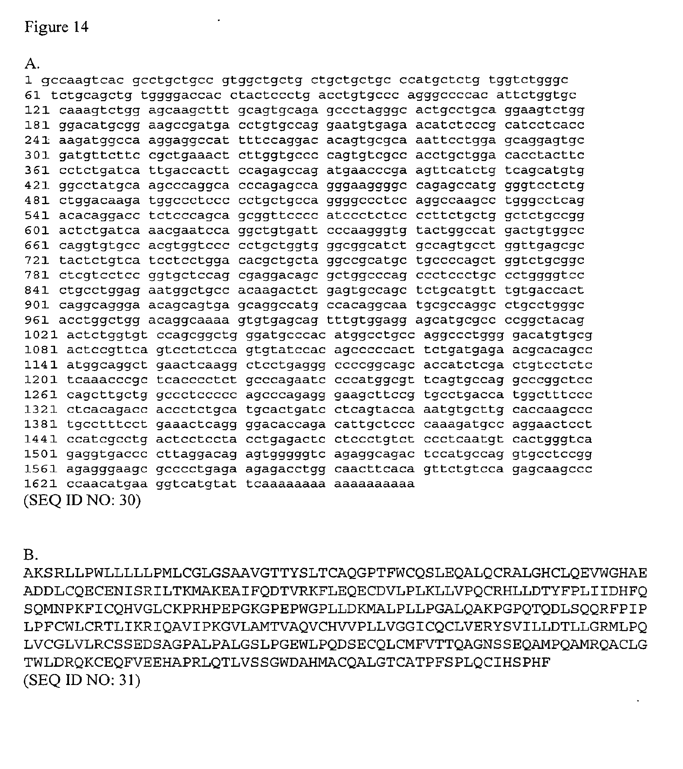

Figure 14A shows the nucleic acid sequence that encodes ovine surfactant protein B (SEQ ID NO: 30). Figure 14B shows the amino acid sequence for ovine surfactant protein B (SEQ ID NO: 31).

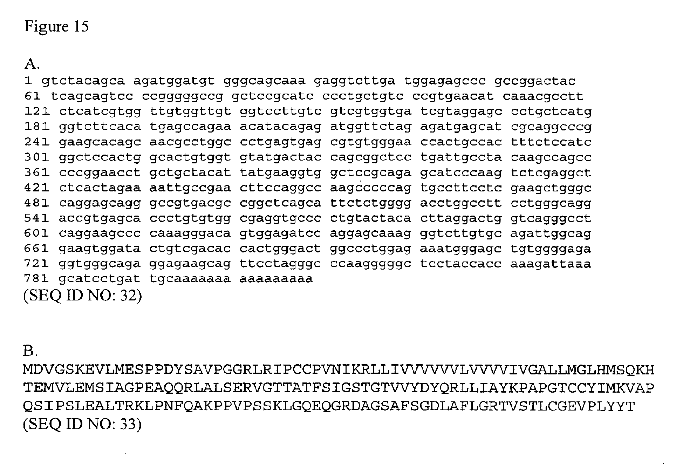

Figure 15A shows the nucleic acid sequence that encodes ovine surfactant protein C (SEQ ID NO: 32). Figure 15B shows the amino acid sequence for ovine surfactant protein C (SEQ ID NO: 33).

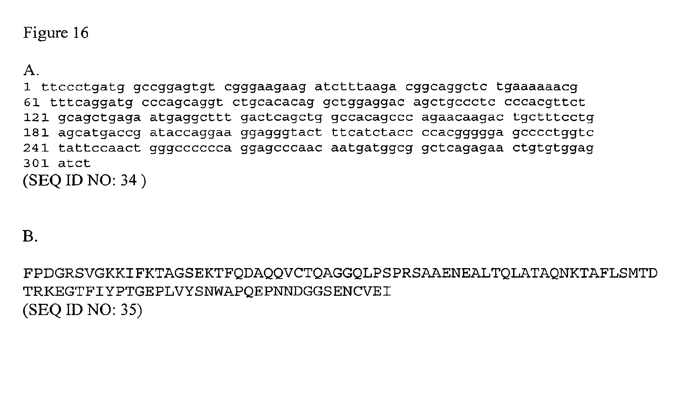

Figure 16A shows the nucleic acid sequence that encodes a partial ovine surfactant protein D (SEQ ID NO: 34). Figure 16B shows a partial amino acid sequence for ovine surfactant protein D (SEQ ID NO: 35).
Figure 17 is a table depicting exemplary human neutrophil elastase (HNE) inhibitors. The reference numbers listed in the table correspond to the compound identifiers referred to in Philip D. Edwards and Peter R. Bernstein in "Synthetic Inhibitors of Elastase," Medicinal Research Reviews, Vol. 14, No. 2, 127-194 (1994).
Figure 18 is a schematic diagram depicting the chemical synthesis of a representative emphysema inhibitor. The emphysema inhibitor as shown contains a glycine linker. The glycine linker (circled) converts the compound to an unnatural amino acid that may be used in a standard peptide synthesis reaction for covalent coupling to the N-terminal 1-25 amino acids of SP-B.

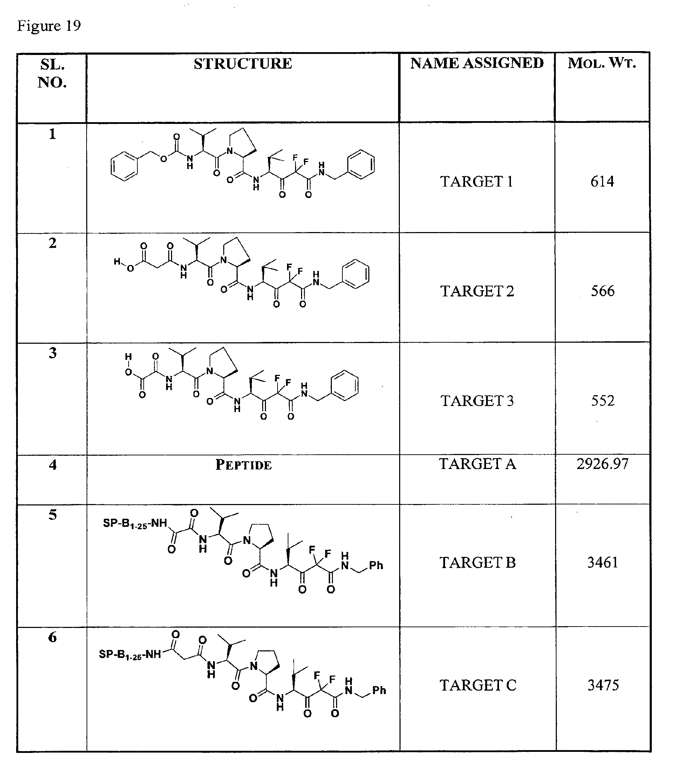

Figure 19 is a table depicting exemplary targets for use as HNE inhibitors. Target 2 attached to the N-terminus of the first 25 residues of the human surfactant B peptide forms target C. Similarly, target 3 attached to the N-terminus of the first 25 residues of the human surfactant B peptide forms target B.

### DETAILED DESCRIPTION

### Surfactant Proteins

The surface active agent comprises at least a portion of a mammalian lung surfactant polypeptide that is substantially non-immunogenic to humans. The polypeptide or portion thereof may be a mammalian lung surfactant moiety or a synthetic mimic thereof. Exemplary surfactant polypeptides may be animal-derived, recombinant, synthetic, analogs, or peptide mimetics.

Natural lung surfactant proteins include SP-A, SP-B, SP-C, SP-D, or portions thereof, alone or in combination with lipids (U.S. Patent No. 5, 302, 581). In some embodiments, the surface active agent comprises the full length surfactant polypeptide. In other embodiments, the surface active agent comprises a portion of a surfactant polypeptide. For example, human SP-B is a 79 amino acid residue polypeptide, however, the N-terminal 25 amino acid residues of SP-B possess therapeutic effects comparable to the whole peptide (Kurutz and Lee, Biochem., 41, 9627-36 (2002)). Exemplary peptides of natural lung surfactant proteins may be at least about 5, 10, 15, 20, 25, 30, 35, 40, 45, or 50 amino acids in length. Exemplary peptides of human SP-B are shown in Table 1.

In one embodiment, the surface active agent comprises human lung surfactant obtained by lung lavage of human cadavers at autopsy or by lung lavage of consenting adults.

In certain embodiments, the surface active agent comprises a non-human mammalian lung surfactant or a fraction thereof. Exemplary non-human surfactants include bovine, porcine or ovine lung surfactants or a fraction thereof. The non-human surfactant may be harvested from the lungs of a non-human mammal using techniques that are well known in the art. For example, porcine surfactant may be obtained from newborn and/or adult pigs harvesting the bronchoalveolar lavage (BAL) of the lungs with saline as described in Bernhard et al., Am. J. Respir. Cell Mol. Biol. 17:41-50 (1997). Harvested BAL fluid is centrifuged to remove cells and then the cell-free BAL fluid is further centrifuged to generate a raw surfactant pellet. Ovine surfactant may be obtained from whole lung lavages of adult sheep as described by Brackenbury et al., Am. J. Respir Cir. Care Med. 163:1135-1142 (2001). The harvested alveolar lavage is centrifuged to remove cellular debris, followed by further centrifugation to obtain a pellet corresponding to a surfactant aggregate pellet. Bovine surfactant may also be obtained from the lung lavages of adult cows as described by Panda et al. (J Colloid Interface Sci., 311:551-5 (2007)). Alveofact ®, a natural bovine surfactant extract containing phospholipids, neutral lipids, SP-B and SP-C polypeptides may also be used.

Proteins and polypeptides derived from or having characteristics similar to those human lung surfactant may also be used. For example, SP-B may be isolated from bovine surfactant using differential organic extraction, column chromatography, and/or preparative SDS-PAGE as described by Beers et al., Am. J. Physiol Lung Cell Mol. Physiol. 262:L773-L778 (1992).

The mammalian lung surfactant polypeptides or portion thereof can also be recombinantly produced. Recombinant SP-A, SP-B, SP-C, SP-D, or a portion thereof is obtainable by expression of a DNA sequence coding for SP-A, SP-B, SP-C, SP-D, or a portion thereof in a suitable prokaryotic or eukaryotic expression system using various known techniques. Recombinant vectors, which are readily adapted to include a isolated nucleic acid encoding a surfactant polypeptide or a portion thereof, host cells containing the recombinant vectors, and methods of making such vectors and host cells as well as using them for the production of the encoded polypeptides by recombinant techniques are well-known. The nucleic acids encoding a surfactant polypeptide or a portion thereof may be provided in an expression vector comprising a nucleotide sequence encoding a surfactant polypeptide that is operably linked to at least one regulatory sequence. It should be understood that the design of the expression vector may depend on such factors as the choice of the host cell to be transformed and/or the type of protein desired to be expressed. The vector copy number, the ability to control that copy number, and the expression of any other protein encoded by the vector, such as antibiotic markers, should be considered. The subject nucleic acids may be used to cause expression and over-expression of a kinase or phosphatase polypeptide in cells propagated in culture, e.g. to produce proteins or polypeptides, including fusion proteins or polypeptides.

Host cells may be transfected with a recombinant gene in order to express a surfactant polypeptide or portion thereof. The host cell may be any prokaryotic or eukaryotic cell. For example, a polypeptide may be expressed in bacterial cells, such as E. coli, insect cells (baculovirus), yeast, or mammalian cells. In those instances when the host cell is human, it may or may not be in a live subject. Other suitable host cells are known to those skilled in the art. Additionally, the host cell may be supplemented with tRNA molecules not typically found in the host so as to optimize expression of the polypeptide. Other methods suitable for maximizing expression of the polypeptide will be known to those in the art.

Methods of producing polypeptides are well-known in the art. For example, a host cell transfected with an expression vector encoding a surfactant polypeptide or portion thereof may be cultured under appropriate conditions to allow expression of the polypeptide to occur. The polypeptide may be secreted and isolated from a mixture of cells and medium containing the polypeptide. Alternatively, the polypeptide may be retained cytoplasmically. Cells are then harvested, lysed, and the protein is isolated from the cell lysates.

A cell culture includes host cells, media, and other by-products. Suitable media for cell culture are well known in the art. The polypeptide may be isolated from cell culture medium, host cells, or both using techniques known in the art for purifying proteins, including ammonium sulfate or ethanol precipitation, acid extraction, anion or cation exchange chromatography, gel filtration chromatography, phosphocellulose chromatography, hydrophobic interaction chromatography, affinity chromatography, hydroxylapatite chromatography, lectin chromatography, ultrafiltration, electrophoresis, immunoaffinity purification with antibodies specific for particular epitopes of a polypeptide of the invention, and high performance liquid chromatography ("HPLC") is employed for purification. Thus, a nucleotide sequence encoding all or a selected portion of a surfactant polypeptide may be used to produce a recombinant form of the protein via microbial or eukaryotic cellular processes. Ligating the sequence into a polynucleotide construct, such as an expression vector, and transforming or transfecting into hosts, either eukaryotic (yeast, avian, insect or mammalian) or prokaryotic (bacterial cells), are standard procedures. Similar procedures, or modifications thereof, may be employed to prepare recombinant polypeptides of the invention by microbial means or tissue-culture technology.

Expression vehicles for production of a recombinant protein include plasmids and other vectors. For instance, suitable vectors for the expression of a polypeptide of the invention include plasmids of the types: pBR322-derived plasmids, pEMBL-derived plasmids, pEX-derived plasmids, pBTac-derived plasmids and pUC-derived plasmids for expression in prokaryotic cells, such as E. coli.

In certain embodiments, mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-I), or Epstein-Barr virus (pHEBo, pREP-derived and p205) can be used for transient expression of proteins in eukaryotic cells. The various methods employed in the preparation of the plasmids and transformation of host organisms are well known in the art. For other suitable expression systems for both prokaryotic and eukaryotic cells, as well as general recombinant procedures, see Molecular Cloning A Laboratory Manual, 2nd Ed., ed. by Sambrook, Fritsch and Maniatis (Cold Spring Harbor Laboratory Press, 1989) Chapters 16 and 17. In some instances, it may be desirable to express the recombinant protein by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the [beta]-gal containing pBlueBac **III**).

In another embodiment, protein production may be achieved using in vitro translation systems. In vitro translation systems are, generally, a translation system which is a cell-free extract containing at least the minimum elements necessary for translation of an RNA molecule into a protein. An in vitro translation system typically comprises at least ribosomes, tRNAs, initiator methionyl-tRNAMet, proteins or complexes involved in translation, e.g., eIF2, eIF3, the cap-binding (CB) complex, comprising the cap-binding protein (CBP) and eukaryotic initiation factor 4F (eIF4F). A variety of in vitro translation systems are well known in the art and include commercially available kits. Examples of in vitro translation systems include eukaryotic lysates, such as rabbit reticulocyte lysates, rabbit oocyte lysates, human cell lysates, insect cell lysates and wheat germ extracts. Lysates are commercially available from manufacturers such as Promega Corp., Madison, Wis.; Stratagene, La Jolla, Calif.; Amersham, Arlington Heights, IU.; and GIBCOBRL, Grand Island, N. Y. In vitro translation systems typically comprise macromolecules, such as enzymes, translation, initiation and elongation factors, chemical reagents, and ribosomes. In addition, an in vitro transcription system may be used. Such systems typically comprise at least an RNA polymerase holoenzyme, ribonucleotides and any necessary transcription initiation, elongation and termination factors. In vitro transcription and translation may be coupled in a one-pot reaction to produce proteins from one or more isolated DNAs. When expression of a carboxy terminal fragment of a polypeptide is desired, i.e. a truncation mutant, it may be necessary to add a start codon (ATG) to the oligonucleotide fragment containing the desired sequence to be expressed. It is well known in the art that a methionine at the N-terminal position may be enzymatically cleaved by the use of the enzyme methionine aminopeptidase (MAP). MAP has been cloned from E. coli (Ben- Bassat et al., (1987) J Bacteriol. 169:751-757) and Salmonella typhimurium and its in vitro activity has been demonstrated on recombinant proteins (Miller et al., (1987) PNAS USA 54:2718-1722). Therefore, removal of an N-terminal methionine, if desired, may be achieved either in vivo by expressing such recombinant polypeptides in a host which produces MAP (e.g., E. coli or CM89 or S. cerevisiae), or in vitro by use of purified MAP (e.g., procedure of Miller et al).

Polypeptides of the invention may also be subject to various changes, such as insertions, deletions, and substitutions, either conservative or non-conservative, where such changes provide for certain advantages in their use. Conservative substitutions are those in which one amino acid residue is replaced by another, biologically similar residue. Examples of conservative substitutions include the substitution of one hydrophobic residue such as isoleucine, valine, leucine or methionine for another, or the substitution of one polar residue for another such as between arginine and lysine, between glutamic and aspartic acids or between glutamine and asparagine and the like. The term "conservative substitution" also includes the use of a substituted amino acid in place of an unsubstituted parent amino acid provided that such a polypeptide also displays the requisite binding activity.

Polypeptides of the invention may also be truncated relative to the full-length mature polypeptide. Polypeptides may be truncated at either the amino-terminus, carboxy-terminus, or both termini. Polypeptides may be truncated by at least one amino acid, or at least 5, 10, 15, 20, 25, 30, 35, 40, 45, 50, 55, 60, 65 or 70 amino acids.

A mammalian lung surfactant polypeptide or a portion thereof can be synthesized from amino acids by techniques that are known to those skilled in the polypeptide art. A summary of the many techniques available may be found in J. M. Steward and J. D. Young, "Solid Phase Peptide Synthesis", W. H. Freeman Co., San Francisco, 1969, and J. Meienhofer, "Hormonal Proteins and Peptides", Vol. 2, p. 46, Academic Press (New York), 1983 for solid phase peptide synthesis, and E. Schroder and K. Kubke, "The Peptides", Vol. 1, Academic Press (New York), 1965 for classical solution synthesis.

In general, these methods comprise the sequential addition of one or more amino acid residues or suitably protected amino acid residues to a growing peptide chain. Normally, either the amino or carboxyl group of the first amino acid residue is protected by a suitable, selectively removable protecting group. A different, selectively removable protecting group is utilized for amino acids containing a reactive side group (e.g., lysine).

Using a solid phase synthesis as an example, the protected or derivatized amino acid is attached to an inert solid support through its unprotected carboxyl or amino group. The protecting group of the amino or carboxyl group is then selectively removed and the next amino acid in the sequence having the complementary (amino or carboxyl) group suitably protected is admixed and reacted under conditions suitable for forming the amide linkage with the residue already attached to the solid support. The protecting group of the amino or carboxyl group is then removed from this newly added amino acid residue, and the next amino acid (suitably protected) is then added, and so forth. After all the desired amino acids have been linked in the proper sequence, any remaining terminal and side group protecting groups (and any solid support) are removed sequentially or concurrently, to afford the final polypeptide. That polypeptide is then washed by dissolving in a lower aliphatic alcohol, and dried. The dried surfactant polypeptide can be further purified by known techniques, if desired.

In certain embodiments, commonly used methods such as t-BOC or f-MOC protection of alpha-amino groups can be used. Both methods involve stepwise syntheses whereby a single amino acid is added at each step starting from the C terminus of the peptide (See, Coligan et al., Current Protocols in Immunology, Wiley Interscience, 1991, Unit 9). Peptides of the invention can be synthesized, for example, by the well known solid phase peptide synthesis methods described in Merrifield, J. Am. Chem. Soc. 85: 2149, 1962, and Stewart and Young, 1969, Solid Phase Peptides Synthesis, pp. 27-62, using a copoly(styrenedivinylbenzene) containing 0.1-1.0 mMol amines/g polymer. On completion of chemical synthesis, the peptides can be deprotected and cleaved from the polymer by treatment with liquid HF-10% anisole for about 1/4-1 hours at 0°C. After evaporation of the reagents, the peptides are extracted from the polymer with 1% acetic acid solution which is then lyophilized to yield the crude material. This can normally be purified by such techniques as gel filtration on Sephadex G-15 using 5% acetic acid as a solvent. Lyophilization of appropriate fractions of the column will yield the homogeneous peptide or peptide derivatives, which can then be characterized by such standard techniques as amino acid analysis, thin layer chromatography, high performance liquid chromatography, ultraviolet absorption spectroscopy, molar rotation, solubility, and quantitated by the solid phase Edman degradation.

In one embodiment, recombinant and/or synthetic SP-B peptides contain amino acids 2, 4, 6, and 9 of SEQ ID NO:5. Prolines 2, 4, and 6 and tryptophan 9 of SEQ ID NO:5 may constitute essential structural motifs for protein function. In some embodiments, SP-B peptides may be substituted at any amino acid residue other than tryptophan 9 amino acid (relative to SEQ ID NO:5).

A lung surfactant polypeptide mimic is generally a polypeptide that is engineered to mimic the essential attributes of human surfactant protein. An exemplary mimetic peptide mimics SP-B. One example of a SP-B mimic is KL4, a 21 amino acid residue peptide comprising the sequence KLLLLKLLLLKLLLLKLLLLK (SEQ ID NO: 94). This SP-B mimetic protein is also known as Lucinactant (Surfaxin®, Discovery Laboratories).

### Surfactant Lipids

In certain embodiments, a surface active agent for use in the invention comprises a surfactant protein, a portion thereof, or a mixture thereof, which associates with natural surfactant lipids *in vivo.* In other embodiments, a surface active agent for use in the invention comprises a lipid or a lipid-protein complex.

Natural mammalian lung surfactant is a complex of phospholipids, neutral phospholipids, and proteins. Surface active agent for use in the invention disclosed herein may comprise one or more lipids. In some embodiments, the surface active agent can comprise, for example, from as little as about 0.05 to 100% weight percent lipid, so long as the resulting composition has surfactant activity. By weight percent is meant the percentage of a compound by weight in a composition by weight. Thus, a composition having 50 weight percent lipid contains, for example, 50 grams lipids per 100 grams total composition. A surface active agent may contain 0.1 to 50 weight percent lipid, although higher concentrations of lipid can be used. Surface active agents containing both phospholipid and a surfactant polypeptide or portion thereof can contain, therefore, 0.1, 1, 10, 50, 80, to almost 100 weight percent lipid and about 50, 20, 10, to less than 1 weight percent surfactant polypeptide. Alternatively, surface active agents may contain the reverse ratios of lipid to surfactant polypeptide.

The term "lipid" as used herein refers to a naturally occurring, synthetic or semi-synthetic (i.e., modified natural) compound which is generally amphipathic. The lipids typically comprise a hydrophilic component and a hydrophobic component. Exemplary lipids include, but are not limited, phospholipids, fatty acids, fatty alcohols, neutral fats, phosphatides, oils, glycolipids, aliphatic alcohols, waxes, terpenes and steroids. The phrase semi-synthetic (or modified natural) denotes a natural compound that has been chemically modified in some fashion.

Examples of phospholipids include native and/or synthetic phospholipids. Phospholipids that can be used include, but are not limited to, phosphatidylcholines (saturated and unsaturated), phospatidylglycerols, phosphatidylethanolamines, phosphatidylserines, phosphatidic acids, phosphatidylinositols, sphingolipids, diacylglycerides, cardiolipin, ceramides, cerebrosides and the like. Exemplary phospholipids include, but are not limited to, dipalmitoyl phosphatidylcholine (DPPC), dilauryl phosphatidylcholine (DLPC) (C12:0), dimyristoyl phosphatidylcholine (DMPC) (C14:0), distearoyl phosphatidylcholine (DSPC), diphytanoyl phosphatidylcholine, nonadecanoyl phosphatidylcholine, arachidoyl phosphatidylcholine, dioleoyl phosphatidylcholine (DOPC) (C18:1), dipalmitoleoyl phosphatidylcholine (C16:1), linoleoyl phosphatidylcholine (C18:2), myristoyl palmitoyl phosphatidylcholine (MPPC), steroyl myristoyl phosphatidylcholine (SMPC), steroyl palmitoyl phosphatidylcholine (SPPC), palmitoyloleoyl phosphatidylcholine (POPC), palmitoyl palmitooleoyl phosphatidylcholine (PPoPC), dipalmitoyl phosphatidylethanolamine (DPPE), palmitoyloleoyl phosphatidylethanolamine (POPE), dioleoylphosphatidylethanolamine (DOPE), dimyristoyl phosphatidylethanolamine (DMPE), distearoyl phosphatidylethanolamine (DSPE), dioleoyl phosphatidylglycerol (DOPG), palmitoyloleoyl phosphatidylglycerol (POPG), dipalmitoyl phosphatidylglycerol (DPPG), dimyristoyl phosphatidylglycerol (DMPG), distearoyl phosphatidylglycerol (DSPG), dimyristoylphosphatidylserine (DMPS), distearoylphosphatidylserine (DSPS), palmitoyloleoyl phosphatidylserine (POPS), soybean lecithin, egg yolk lecithin, sphingomyelin, phosphatidylinositols, diphosphatidylglycerol, phosphatidylethanolamine, phosphatidic acids, and egg phosphatidylcholine (EPC).

Examples of fatty acids and fatty alcohols include, but are not limited to, sterols, palmitic acid, cetyl alcohol, lauric acid, myristic acid, stearic acid, phytanic acid, dipamlitic acid, and the like. Exemplary fatty acids include palmitic acid.

Examples of fatty acid esters include, but are not limited to, methyl palmitate, ethyl palmitate, isopropyl palmitate, cholesteryl palmitate, palmityl palmitate sodium palmitate, potassium palmitate, tripalmitin, and the like.

Surfactant polypeptide and surfactant lipids interact by hydrostatic interactions. Charged amino acids interact with the lipid polar head groups and hydrophobic amino acids interact with phospholipid acyl side chains. For example, SP-B and SP-C are hydrophobic proteins. Both SP-B and SP-C preferentially bind anionic lipids, such as phosphatidylglycerol (PG), and not DPPC. SP-A and SP-D are hydrophilic proteins and interact with a broad range of amphipathic lipids, including glycerophospholipids, sphingophospholipids, glycosphingolipids, lipid A, and lipoglycans. SP-A binds DPPC. By way of example, hydrostatic interactions are observed with the SP-B mimetic, KL4, and lipids in natural surfactant or lipids comprised in the surface active agent. For example, the lysine residues in the KL4 peptide interact with the charge head groups of DPPC and the hydrophobic leucine resides interact with the phospholipid acyl side chains of phosphatidylglycerol.

In certain embodiments, a drug composition as disclosed herein comprises a surface active agent comprising a portion of a mammalian lung surfactant polypeptide or mimic thereof and does not additionally comprise a lipid or a mixture of lipids. Drug compositions administered by inhalation comprising surface active agents comprising only a portion of a mammalian lung surfactant polypeptide or mimic thereof can interact with natural surfactant in the lungs through hydrostatic interactions. For example, recombinant SP-B can interact with natural surfactant in the lungs by binding anionic phospholipids, such as phosphatidylglycerol.

In other embodiments, a drug composition as disclosed herein comprises a surface active agent comprising both a portion of a mammalian lung surfactant polypeptide or a mimic thereof and at least one lipid. To facilitate absorption of drug compositions comprising both a polypeptide or mimic thereof and at least one lipid into natural surfactant in the lungs, phopholipid monolayers mimicking those found in natural surfactant can be used. Exemplary lipid mixtures include dipalmitoylphosphatidylcholine/palmitoyloleoylphosphatidylglycerol, for example at a 7:3 w/w ratio. The mammalian lung surfactant polypeptide can be inserted into the phosphoplipid monolayer and the protein/lipid mix can be absorbed into the natural surfactant at the alveolar/gas interface in the lungs following inhalation.

### Pulmonary Active Drug

Pulmonary active drugs may include, but are not limited to elastase inhibitors, corticosteroids, bronchodilators, antibiotics, and chemotherapeutic acids.

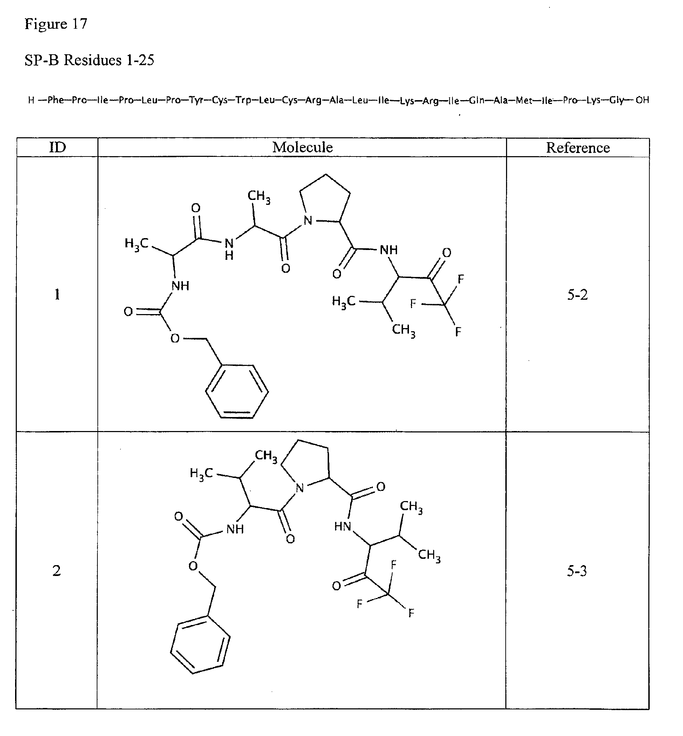

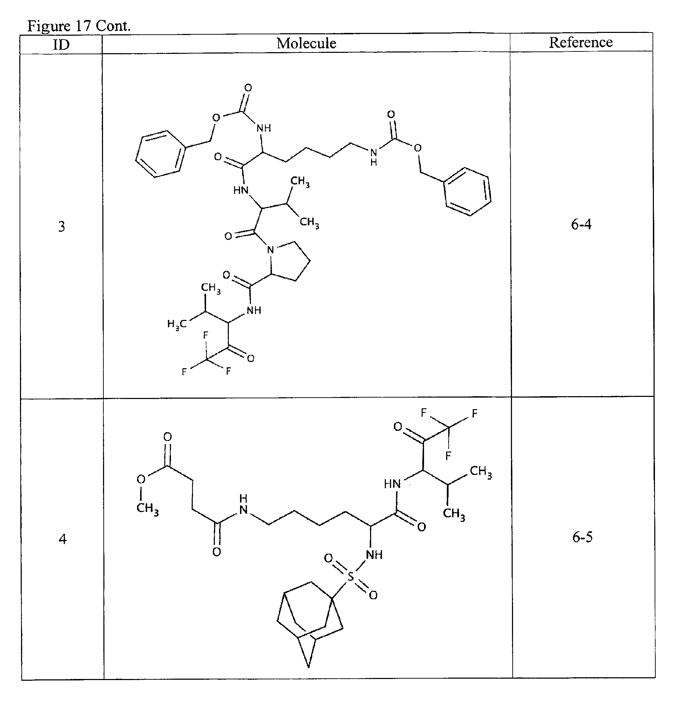

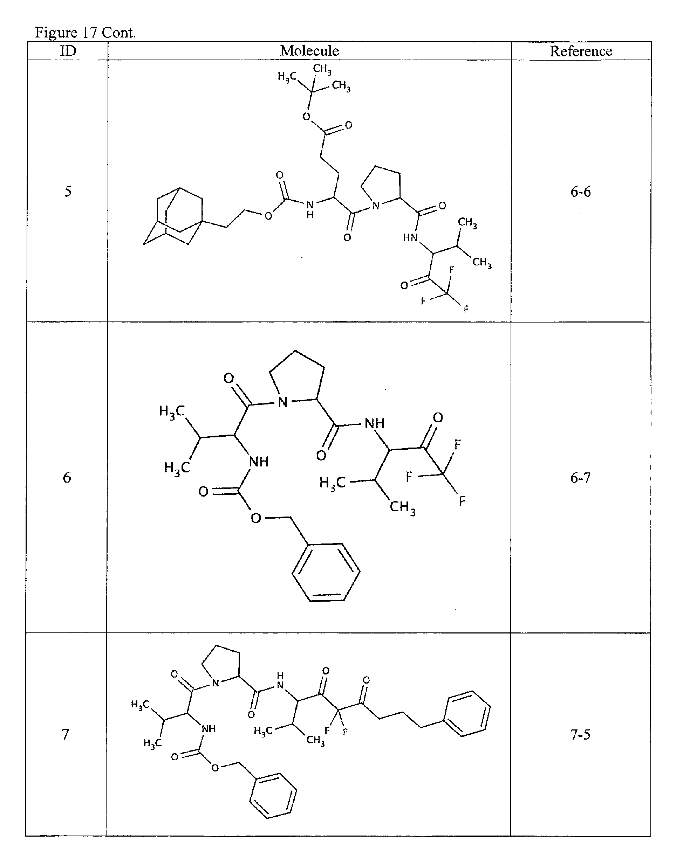

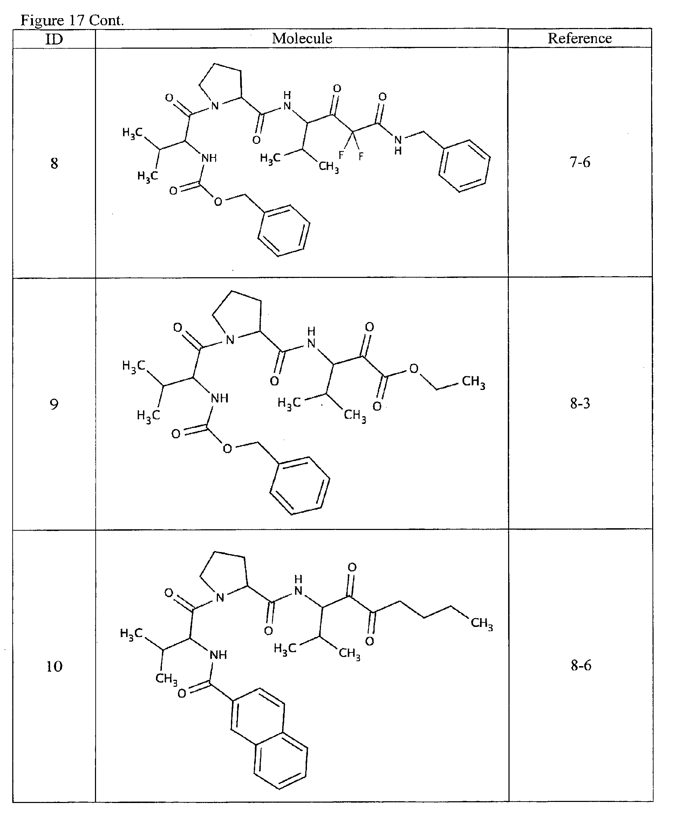

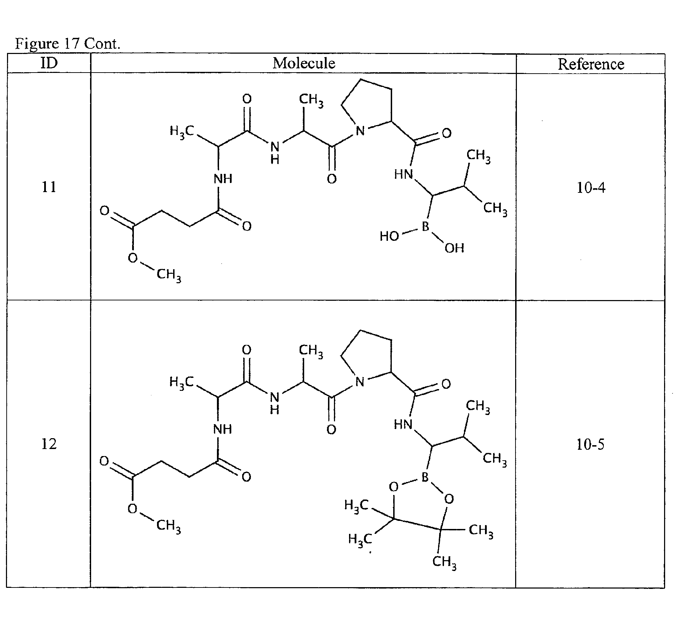

Exemplary elastase inhibitors include the compounds shown in Figure 17. Other exemplary elastase inhibitors include the compounds described by Philip D. Edwards and Peter R. Bernstein in "Synthetic Inhibitors of Elastase," Medicinal Research Reviews, Vol. 14, No. 2, 127-194 (1994), which is incorporated herein by reference. Two or more elastase inhibitors may be linked to a surface active agent of the invention and administered in combination. Alternatively, an elastase inhibitor may be linked to a surface active agent and administered in combination with a second elastase inhibitor linked to a surface active agent.

Exemplary corticosteroids that may be delivered to the lung include, but are not limited to, alclometasone, aldosterone, amcinonide, beclometasone, betamethasone, budesonide, ciclesonide, clobetasol, clobetasone, clocortolone, cloprednol, cortisone, cortivazol, deflazacort, deoxycorticosterone, desonide, desoximetasone, desoxycortone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluprednidene, fluticasone, formocortal, halcinonide, halometasone, hydrocortisone/cortisol, hydrocortisone aceponate, hydrocortisone buteprate, hydrocortisone butyrate, loteprednol, medrysone, meprednisone, methylprednisolone, methylprednisolone aceponate, mometasone furoate, paramethasone, prednicarbate, prednisone, prednisolone, prednylidene, rimexolone, tixocortol, triamcinolone, and ulobetasol. Two or more corticosteroids may be linked to a surface active agent of the invention and administered in combination. Alternatively, a corticosteroid may be linked to a surface active agent and administered in combination with a second corticosteroid linked to a surface active agent.

Pulmonary active drugs may also include, bronchodilators such as short-acting, β2-adrenergic receptor agonists, long-acting β2-adrenergic receptor agonists, short-acting anti-cholinergics, and long-acting anti-cholinergics. Non-limiting short-acting, β2-adrenergic receptor agonists include salbutamol or albuterol, terbutaline, fenoterol, fenoterol hydrobromide, rimiterol, reproterol, pirbuterol, isoprenaline, orciprenaline, bitolterol, and broxaterol. Non-limiting long-acting β2-adrenergic receptor agonists include salmeterol, salmeterol xinafoate, formoterol, formoterol fumarate, clenbuterol, and procaterol. Non-limiting short-acting anti-cholinergics include ipratropium, ipratropium bromide, oxitropium and its salts. Non-limiting long-acting anti-cholinergics include tiotropium and tiotropium bromide monohydrate. Other bronchodialators may include, but are not limited to, aminophyliline, iorciprenaline, oxtriphylline, terbutaline sulfate, and theophylline. Two or more bronchodilators may be linked to a surface active agent of the invention and administered in combination. Alternatively, a bronchodilator may be linked to a surface active agent and administered in combination with a second bronchodilator linked to a surface active agent.

Exemplary antibiotics that may be delivered to the lung include, but are not limited to penicillins, penicillins and beta-lactamase inhibitors, cephalosporins (generation I, II, III, and IV), macrolides and lincosamines, quinolones and fluoroquinoloes, carbepems, monbactams, aminoglycosides, glycopeptides, tetracylines, sulfonamides, rifampin, oxazolidones, streptogramins, sulfanomides, and others. Two or more antibiotics may be linked to a surface active agent of the invention and administered in combination. Alternatively, an antibiotic may be linked to a surface active agent and administered in combination with a second antibiotic linked to a surface active agent.

Exemplary penicillins include, but are not limited to, amoxicillin, ampicillin, bacampicillin, carbenicillin, carbenicillin indanyl, mezlocilin, piperacillin, and ticarcilin.

Exemplary penicilins and beta-lactamase inhibitors include, but are not limited to, amoxicillin-clavulanic acid, ampicillin-sulbactam, benzylpenicillin, cloxacilin, dicloxacilin, phenoxymethylpenicillin, carbenicillin, methicillin, oxacilin, penicillin G (benzathine, potassium, procaine), penicilin V, propicillin, epicillin, cyclacillin, piperacilin plus tazobactam, ticarcilllin plus clavulanic acid, and naficillin.

Exemplary cephalosporins (generation I) include, but are not limited to, cefadroxil, cefazolin, cephalexin, cephalothin, cephapirin, and cephradine. Cephalosporins (generation **II**) include, but are not limited to, cefaclor, cefamandole, cefoicid, ceforanide,cefoxitin, cefprozil, ceftmetazole, cefuroxime, cefuoxime axetil, and loracarbef. Cephalosporins (generation **III**) include, but are not limited to, cefdinir, ceftibuten, cefditoren, cefatamet, cefoperazone, cefixime, cefotaxime, cefpodoxime, ceftazidime, ceftibuten, ceftizoxime, and ceftriaxone. Cephalosporins (generation IV) include, but are not limited to, cefepime.

Exemplary marcolides and lincosamine include, but are not limited to, azithromycin, clarithromycin, clindamycin, dirithromycin, erythromycin, lincomycin, telithromycine, and troleandomycin.

Monobactams include, but are not limited to, include aztreonam. Carbepenems include, but are not limited to, doripenem, imipenem-cilastatin, and meropenem.

Aminoglycosides include, but are not limited to, amikacin, amikacin sulfate, gentamicin, genatmicin sulfate, kanamycin, metilmicin, neomycin, netilmicin, streptomycin, tobramycin, and paromycin.

Glycopeptides include, but are not limited to, dalbavancin, oritavancin, telavancin, teicoplanin, and vancomycin.

Tetracylines include, but are not limited to, demclocylline, doxycycline, methacyline, minocyline, oxytetracycline, tetracyline, and chloretracycline.

Oxazolidonones include, but are not limited to linezolid. Streptogramins include but are not limited to quinoprisitin plus dalfopristin.

Sulfonamides include, but are not limited to mafenide, silver sulfadiazine, sulfacetamide, sulfadiazine, sulfamethoxazole, sulfasalzine, sulfanilamide, sulfisoxazole, trimethoprim-sulfamethoxazole, and sulfamethizole.

Other antibiotics include, but are not limited to, bacitracin, chloramphenical, Colistemetate, Fosfomycin, Isoniazid, Methenamine, Metronidazol, Mupirocin, Nitrofurantoin, Nitrofurazone, Novobiocin, Polymyxin B, Spectinomycin, Trimethoprim, Colistin, Cycloserine, Capreomycin, Pyrazinamide, Para-aminosalicyclic acid, Erythromycin ethylsuccinate plus sulfisoxazole, and tigecycline.

Chemotherapeutic drugs that may delivered to the lung include, but are not limited to, alkylating agents, antiestrogens, aclarubicin, actinomycin D, aldesleukin, alemtuzumab, alitretinoin, allopurinol, altretamine, amifostine, anastrozole, asparaginase, bexarotene, bisantrene, bleomycin, busulfan, BCNU (carmustine), calusterone, capecitabine, carboplatin, celecoxib, chlorambucil, cisplatin, cladribine, cyclophosphamide, cyclooxygenase-2 inhibitor, cytarabine, CCNU (lomustine), dacarbazine, daunorubine, daunomycin, denileukin diftitox, dexrazoxane, diaziquone, docetaxel, doxorubicin, epirubicin, epoetin alfa, esorubicin. estramustine, etoposide (VP-16), exemestane, Filgrastim, floxuridine, fludarabine, 5-fluorouracil, fulvestrant, galactitol, gemcitabine, gemtuzumab, goserelin acetate, hydroxyurea, ibritumomab tiuxetan, idarubicin, ifosfamide, imatinib mesylate, interferon alpha, interferon gamma, iriniotecan, iroplatin, letrozole, leucovorin, levamisole, lonidamine, megrestrol acetate, melphalan, mercaptopurine, mesna, methotrexate, methoxsalen, mitomycin C, mitotane, mitoxantrone, mitoguazone, nandrolone phenpropionate, Nofetumomab, nitrogen mustard, oprelvekin, oxaliplatin, paclitaxel, pamidronate, pegademase, pegaspargase, pegfilgrastim, pentostatin, pipobroman, plicamycin, porfimer sodium, procarbazine, progestins, prednimustine, PCNU, quinacrine, rasburicase, rituximab, sargramostim, streptozocin, talc, tamoxifen, temozolomide, teniposide (VM-26), testolactone, thioguanine, thiotepa, topotecan, toremifene, tositumomab, trastuzumab, tertinoin, uracil mustard, valrubicin, vinblastine, vincristine, vindesine, vinorelbine, and zoledronate.

Two or more chemotherapeutic agents may be linked to a surface active agent of the invention and administered in combination. Alternatively, a chemotherapeutic agent may be linked to a surface active agent and administered in combination with a second chemotherapeutic agent linked to a surface active agent.Exemplary combination therapies include paclitaxel and carboplatin, cisplatin and vinorelbline tartrate, cisplatin and etoposide, and carboplatin and etoposide.

### Covalent Linkage

Many strategies can be used to covalently link a pulmonary active drug to a surface active agent for use in the invention. Generally, the pulmonary active drug can be attached covalently to the surface active agent using a bond or a linker that preserves the native site of the drug and retains significant dwell time of the surface active agent at the lung/air interface. At least one additional residue can be added at the amino- or carboxy- terminus or at an internal amino acid residue of a surfactant polypeptide disclosed herein to generate a linker for covalently bonding a drug molecule to the surface active agent. In an exemplary embodiment, SP-B is extended by at least one amino acid to create an unnatural amino acid by automated peptide synthesis. A drug can be conjugated to the amino acid, e.g., glycine residue through an amino- or hydroxyl- group. Representative covalent linkages could include an ester, an amide, or urea. (March, Advanced Organic Chemistry, 4th Ed., John Wiley & Sons, 1992.)

Amino acid residue linkers are usually at least one residue and can be 40 or more residues, more often 1 to 10 residues, and most often 1 to 5 amino acid residues in length. The linker is usually a small neutral polar or non-polar amino acid. Typical amino acid residues used for linking are glycine, tyrosine, cysteine, lysine, glutamic acid, and aspartic acid, or the like.

In other embodiments, the linker may be a heterobifunctional linker that is not a naturally occurring amino acid.

### Methods of Administration

Compositions of the invention are delivered to the lungs by inhalation. Inhalation devices, such as inhalers (including dry powder inhaler and metered dose inhalers) and nebulizers (also known as atomizers) may be used to deliver the disclosed compositions to the lungs. Exemplary dry powder inhalers can be obtained from Inhale Therapeutic Systems as described in U.S. Pat. Nos. 5,458,135; 5,740,794; 5,785,049. Dry powder inhalers can also be obtained from 3M as described in U.S. Patent 6,029,661.

The compositions disclosed herein may also be administered using a metered dose inhaler (MDI) containing a solution or suspension of drug in a pharmaceutically inert liquid propellant, e.g., a chlorofluorocarbon (CFC) or fluorocarbon, as described in U.S. Pat. No. 5,320,094 and U.S. Pat. No. 5,672,581. Metered dose inhalers are designed to deliver a fixed unit dosage of medicament per actuation or "puff", for example in the range of 10 to 5000 microgram medicament per puff. Exemplary metered dose inhibitors can be obtained from 3M as described in U.S. Patent Nos. 5,224,183; 5,290,534; 5,511,540; 6,454,140; and 6,615,826. Metered dose inhalers may also be CFC-free. Drug compositions to be used with an inhaler may be in the form of aerosolized solid particles or droplets of liquid or suspension.

Alternatively, the compositions described herein may be dissolved or suspended in a solvent, e.g., water or saline, and administered by nebulization. Exemplary nebulizers for delivering an aerosolized solution include the AERx™ (Aradigm), the Ultravent® (Mallinkrodt), the Pari LC Plus™ or the Pari LC Star™ (Pari GmbH, Germany), the DeVilbiss Pulmo-Aide, and the Acorn II® (Marquest Medical Products).

### Drug Formulation

Drug compositions disclosed herein can be formulated into a solution and/or a suspension of particles in a carrier appropriate for inhalation into the respiratory tract and the lungs. Such carriers are also well known to the ordinary artisan familiar with inhalants for the delivery of fine droplets and insufflations for the delivery of inhalable fine particles, on the order of, for example, from about 0.5 to 1 micron, and preferably from about 0.5 to about 0.7 micron, comprised of powders, mists or aerosols, into the respiratory tract as described in Remington's Pharmaceutical Sciences, 16th edition, 1980, Ed. By Arthur Osol, which is incorporated herein by reference.

In one embodiment, drug compositions for inhalation administration can be administered as powders. The powdered drug or composition is normally located within a container such as a hard gelatin capsule or a blister package, or a multi-dose devise. The capsule or blister is ruptured or broached within in an inhaler device, thereby enabling the powder to be inhaled. Generally, the mean particle size of the drug used for inhalation is between 1 and 10 micron with the size range between 2 and 5 microns being particularly suitable for penetrating the peripheral airways of the lungs. Such particle size ranges are commonly achieved by micronisation or spray drying.

A powdered drug composition is often administered as a composition comprising a blend or mixture of the medicament with an inert carrier. Usually the inert carrier has a mean particle size substantially larger than that of the drug. This provides, among other advantages, an improvement in the flow properties and dispensing accuracy of the composition.

Commonly described carrier materials for produced drug, include calcium carbonate and sugars, for example sucrose, mannitol or dextrose or, more particularly, lactose, which are pharmaceutically acceptable and pose no problems of toxicity, since any residues imbibed during dosing are well tolerated upon digestion or may be easily eliminated by disillusion (e.g., in the case of the sugars) or mucocilliary clearance from the lung.

The composition in the capsule or blister is frequently about 25 mgs. This weight probably represents the maximum quantity of powder that may be comfortably inhaled without undue side effects, such as coughing, and also corresponds to the minimum quantity that is usually dispensed by filling machines.

In certain embodiments, compositions formulated for powder inhalation comprise a carrier present at a concentration of about 95.0 to 99.99%. More particularly, from 97.0 to 99.9%, especially from 98.0 to 99.8%, by weight. Processes for preparing such powders, by the application or adaptation of known methods, also constitute features of the invention.

In other embodiments, the drug composition may be formulated as an aerosol formulation using methods well known in the art. One widely used method for dispensing such an aerosol formulation involves making a suspension formulation of the drug as a finely divided powder in a liquefied propellant gas. Alternatively a solution formulation can be prepared where the drug is dissolved in a propellant system, perhaps containing solubilizers and co-solvents to aid dissolution of the drug. Pressurized metered dose inhalers (pMDI) are normally used to dispense such formulations to a patient. Propellants may include chlorofluorocarbon (CFC), fluorocarban (FC), or hydrofluroalkane (HFA) propellants.

### Methods of Treatment

The disclosure also provides methods for treating a subject suffering from lung inflammation or lung disease. In another embodiment, the disclosure provides a method for treating a subject who is at risk of suffering from a lung disorder. The method comprises administering to the subject a conjugate comprising a pulmonary active drug covalently bonded to a surface active agent, which has an affinity for the human alveolar/gas interface and which comprises at least a portion of a mammalian lung surfactant polypeptide or a mimic thereof that is substantially non-immunogenic to humans. The conjugate is administered to the subject by inhalation in an amount effective to induce a drug effect in the lungs. The subject may be a human, monkey, chimpanzee, horse, dog, cat, cow, sheep, pig, rat or mouse. In exemplary embodiments, the subject is a human.

The subject in need of treatment is suffering from lung inflammation or is suffering from or at risk of suffering from lung disease. Exemplary lung diseases that may be treated with the drug composition described herein include, but are not limited to, emphysema, chronic bronchitis, chronic obstructive pulmonary disease (COPD), asthma, respiratory distress disorder (RDS), pneumonia, tuberculosis or other bacterial infection, cystic fibrosis, and/or lung cancer.

### Dosage

The administration of pulmonary active drugs conjugated to a surface active agent reduces the dosing frequency relative to administration of an unconjugated drug. In certain embodiments, the administration step may be repeated once daily, every other day, every three days, every four days, every five days, or weekly.

### EXEMPLIFICATION

### Example 1: Conjugation of Human Neutrophil Elastase Inhibitors to a SP-B peptide

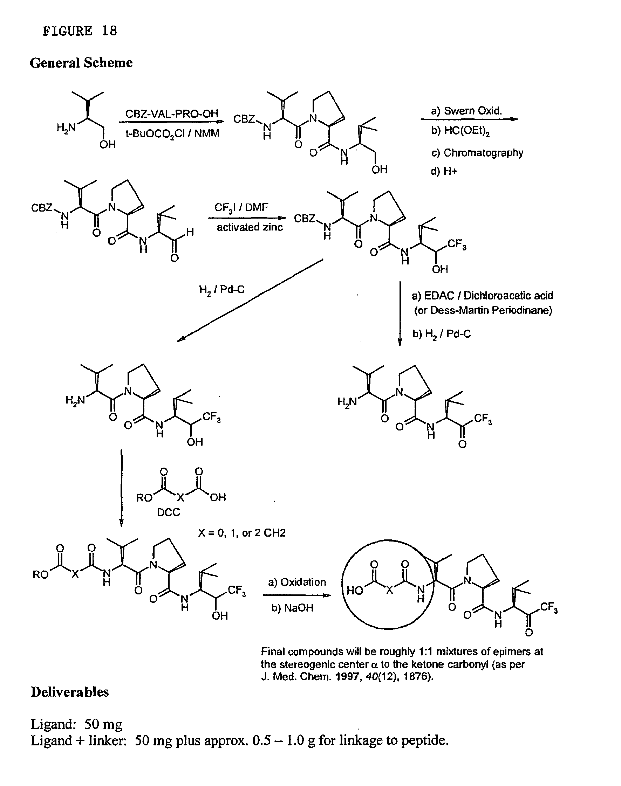

A panel of potent small molecule human neutrophil elastase (HNE) inhibitors can be conjugated to a SP-B peptide comprising the amino terminal 25 amino acids of SP-B (FPIPLPYCWLCRALIKRIQAMIPKG) (SEQ ID NO: 88). The total molecular weight of the SP-B peptide comprising the amino terminal 25 amino acids is 2926.97 after water molecule elimination. The HNE inhibitors are conjugated to the SP-B N-terminal 25-mer using a glycine linker similar to the linkage depicted in Figure 18.

### Example 2: Synthesis of Target B

All solvents used for the reaction were LR grade solvents. Room temperature (RT) indicates temperature ranging from 27-32°C. All the reactions were monitored by TLC unless specified. Solutions were evaporated under reduced pressure using rotary evaporator. NMR was taken on Varian 400 MHz. Column chromatography was done using silica gel 100-200 mesh unless specified.

### Synthesis of Stage 1

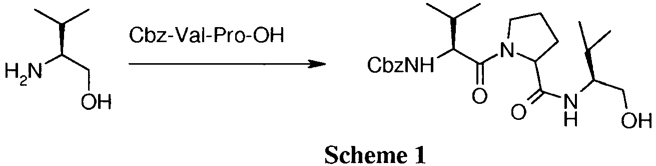

A solution of Cbz-Val-Pro-OH (5 g, 14 mmol) in dry tetrahydrofuran (85 mL) was cooled to -20°C under nitrogen. N-methylmorpholine (1.74 mL, 15 mmol) followed by isobutyl chloroformate (2 mL, 15 mmol) was added to the reaction mixture. The reaction mixture was stirred at -20°C for 15 min and then cooled to -40°C. A solution of L-Valinol (1.62 g, 15 mmol) in tetrahydrofuran (25 mL) was added drop wise to the reaction mixture. The reaction mixture was allowed to warm to room temperature overnight. The reaction mixture was filtered and the filtrate was diluted with ethyl acetate (60 mL). The combined organic layers were washed successively with 1N HCl (60 mL), NaHCO₃ (30 mL) and brine (30 mL). The organic layer was dried over sodium sulphate, filtered and concentrated to give the desired product (5.7 g). HPLC Rt: 5.76; LCMS (M+1): 434; Yield: 92.5%.

### Synthesis of Stage 2

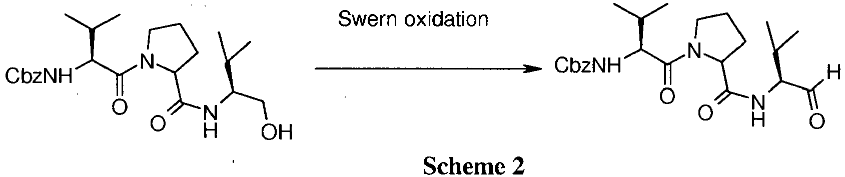

A solution of oxalyl chloride (2.45 mL, 28 mmol) in dry dichloromethane (110 mL) was cooled to -60°C and a solution of DMSO (4.09 mL, 57.7 mmol) in dichloromethane (35 mL) was added drop wise over a period of 1 h, maintaining the reaction mixture temperature at -45°C. The reaction mixture was allowed to warm to -30°C and a solution of stage-1 (6.1 g, 14 mmol) in dichloromethane (35 mL) was added drop wise over a period of 1 h. The reaction mixture was stirred at -25°C for 1h. The reaction mixture was cooled to -40°C and diisopropylethylamine (10mL, 57.7mmol) was added drop wise over a period of 1h. The reaction was warmed to room temperature and then washed with 1 N HCl (60 mL) and brine (60 mL). The organic layer was dried over sodium sulphate, filtered and the filtrate was concentrated under reduced pressure to give the desired product (5.7 g). HPLC Rt: 6.81; LCMS (M+1): 432; Yield: 94%.

### Synthesis of Stage 3

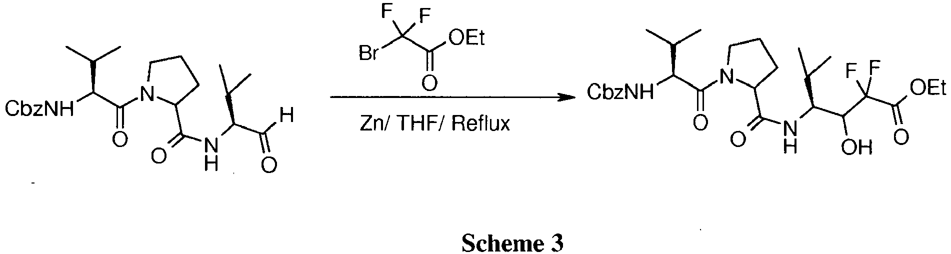

A suspension of Zinc (2.5 g, 39 mmol) and stage-2 (5.64 g, 13 mmol) in dry tetrahydrofuran (100 mL) was heated to 60°C under nitrogen atmosphere. Ethyl bromodifluoroacetate (7.93 g, 39 mmol) was added and the reaction was heated to 60°C for 1 h. The reaction mixture was cooled to room temperature, tetrahydrofuran was removed under reduced pressure and ethyl acetate (100 mL) was added. The reaction mixture was washed with 1M KHSO₄ (50 mL) and brine (50 mL) and dried over sodium sulphate. The organic layer was filtered and concentrated under reduced pressure to give the crude product, which was then purified by preparative HPLC to give the desired product (2.9 g). HPLC Rt: 7.44; LCMS (M+1): 556; Yield:40%.

### Synthesis of Stage 4

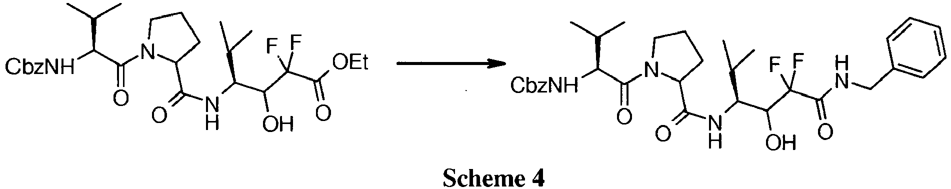

A solution of stage-3 (1.8 g, 3.2 mmol) and benzyl amine (1.06 mL, 9.7 mmol) in ethanol (40 mL) was stirred at reflux for 4 h. The reaction mixture was cooled to room temperature and stirred for an additional 48 h under nitrogen atmosphere. The solvent was removed under reduced pressure. The residue was dissolved in ethyl acetate (50 mL) and the solution was washed with 1N HCl (20 mL) and brine (20 mL). The organic layer was dried over sodium sulphate, filtered and the solvent was removed under vacuum to give the desired product (1.7 g). HPLC Rt: 7.56; LCMS (M+1): 616; Yield: 88%.

### Stage-5

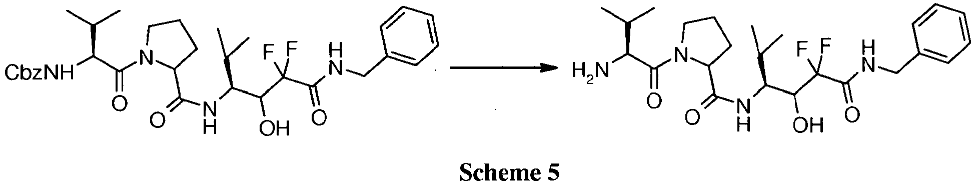

A mixture of stage-4 (1.85 g, 3 mmol) and 20% palladium hydroxide (1 g) in ethyl acetate (50 mL) was placed in a pressure vessel at 200 psi for 20 h. The reaction mixture was filtered through celite and the filtrate was concentrated under vacuum to give the desired product along with some starting material, which was used as such for further reaction without any purification (1.1 g). HPLC Rt: 4.90; LCMS (M+1): 482; Yield: 80%.

### Stage-6

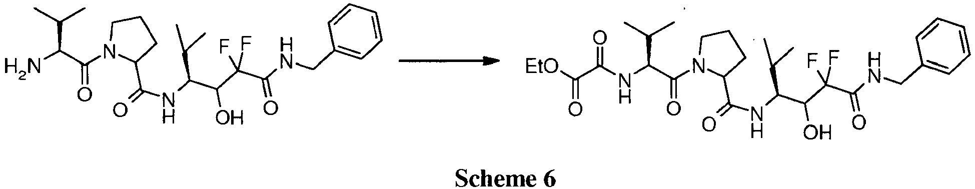

To a solution of stage-5 (1g, 2.1 mmol) in dry dichloromethane (50 mL) at 0 °C was added triethylamine (0.25 mL, 1.8mmol). Then a solution of Ethyl oxalyl chloride (0.2 mL, 1.87 mmol) in dichloromethane (20 mL) was added drop wise. The reaction mixture was allowed to warm to room temperature and stirred for further 2 h. The reaction mixture was quenched with water (25 mL). The organic layer was separated, dried over sodium sulphate and evaporated under reduced pressure to give the desired product, which was then purified by preparative HPLC. HPLC Rt: 6.72; LCMS (M+1): 583; Yield: 0.5 g (42%).

### Stage-7

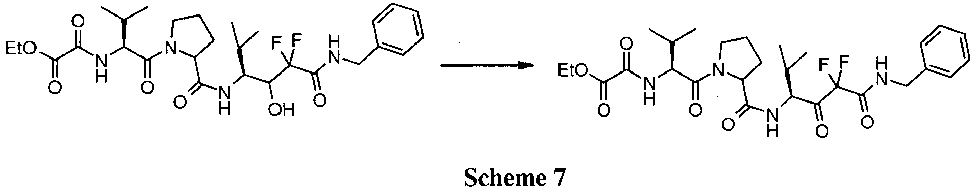

Trifluoroacetic acid (0.92 mL, 12.02 mmol) was added to a stirred solution of stage-6 (1.75 g, 3 mmol) and Dess-Martin periodinane (5.1 g, 12.02 mmol) in dry dichloromethane (25 mL). The mixture was stirred for 4 h at room temperature under nitrogen atmosphere. Ethyl acetate (100 mL) was added and the mixture was washed with saturated sodium thiosulphate (60 mL), saturated NaHCO₃ (60 mL) and brine (60 mL). The organic layer was dried over sodium sulphate, filtered and evaporated under reduced pressure to give the desired product, which was used as such for the further step. HPLC Rt: 7.32; LCMS (M+1): 581; Yield: 1.7 g (97%).

### Stage-8

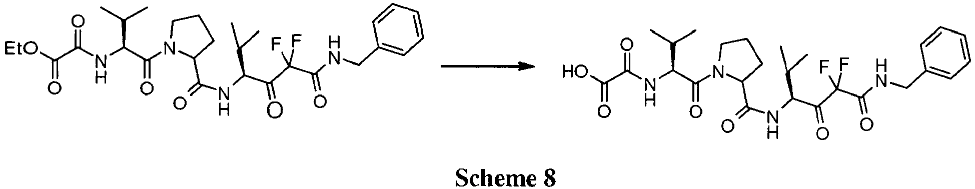

A solution of stage-7 (2.2 g, 3.7 mmol) in methanol water 1:1 (20 mL) was treated with 1N NaOH (0.18 g, 4.5 mmol) and stirred at room temperature for 4 h. The reaction mixture was concentrated to remove methanol, acidified with 1N HCl and extracted with ethyl acetate (60 mL). The organic layer was dried over sodium sulphate and evaporated under reduced pressure to give the desired product, which was then purified by preparative HPLC. HPLC Rt: 6.47-6.61; LCMS (M+1): 553; Yield: 500 mg (24 % After prep).

### Stage-9

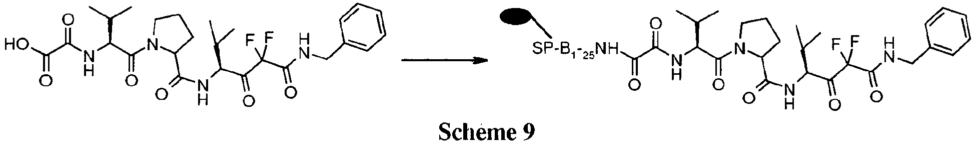

Wang resin (2.5 g, 0.5eq) was treated with 20% piperidine in DMF (15 mL) and stirred for about an hour. It was then washed with DMF (2 times, 15 mL), DCM (3 times, 15 mL) and then dried under vacuum. Kaiser test was performed on the resin to ensure complete removal of F-moc group. Then it was used for coupling with the stage -8 product. To a solution of the resin in DMF was added a solution of the above acid (97 mg, 0.176 mmol) in DMF (5 mL), followed by the addition of PyBoP (91 mg, 0.176 mmol) and N-methyl morpholine (0.24 mL, 0.22 mmol). The reaction mixture was allowed to shake on a shaker for about 3 h. The solution was decanted, fresh lot of the above reagents was again added and it was further allowed to shake for 3 h. This process was repeated for about 4 times. The solution was decanted, and the resin was washed with DMF (3 times, 15 mL), DCM (3 times, 15 mL) dried under vacuum. Kaiser test was performed on the resin to ensure that the coupling has taken place.

### Stage-10

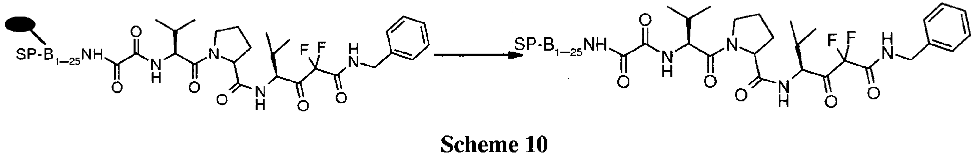

Procedure for making the cleavage solution. TFA (81%), Phenol (5%), Thioanisole (5%), 1,2,Ethanedithiol (2.5%), Water (3%), Dimethylsulphide (2%), ammonium iodide (1.5%). To the resin was added the cleavage solution and was allowed to shake on a shaker for about 3 h. The resin was filtered through cotton and washed with TFA. The filtrate was then concentrated under vacuum and triturated with cold ether to give a white solid, which was then purified by preparative HPLC to give the desired product. LCMS (M/3): 1155; Yield: 46mg (1.84%).

### Example 3: Synthesis of Target C

### Stage-6

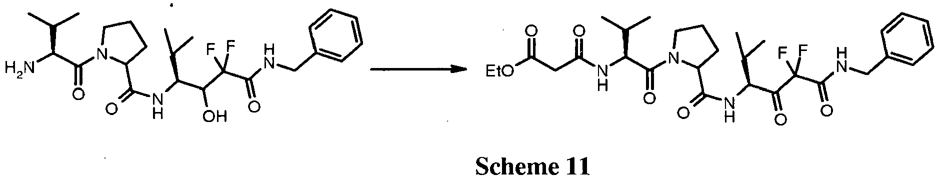

To a solution of stage-5 (3.2 g, 6.6 mmol) in dry THF (50 mL) at room temperature was added mono ethyl malonate (0.7 mL, 6 mmol) followed by HOBT (1.63 g, 12 mmol), EDCI (1.27 g, 6.6 mmol) and N-methyl morpholine (1.6 mL, 15 mmol). The reaction mixture was then allowed to stir at room temperature for 4 h. The reaction mixture was then quenched with water and evaporated under reduced pressure in order to remove THF. The aqueous layer was then extracted with ethyl acetate (100 mL) .The organic layer was dried over sodium sulphate and concentrated to give the crude product which was then purified by preparative HPLC (0.98 g). HPLC Rt: 6.44; LCMS (M+1): 596; Yield: 25% After prep.

### Stage-7

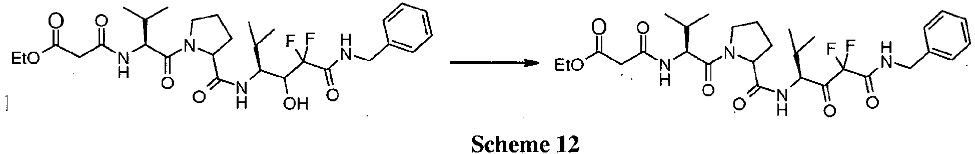

Trifluoroacetic acid (0.45 mL, 5.7 mmol) was added to a stirred solution of stage-6 (0.86 g, 1.45 mmol) and Dess-Martin periodinane (2.46 g, 5.7 mmol) in dry dichloromethane (30 mL). The mixture was stirred for 4 h at room temperature under nitrogen atmosphere. Ethyl acetate (50 mL) was added and the mixture was washed with saturated sodium thiosulphate (20 mL), saturated NaHCO₃ (20 mL) and brine (20 mL). The organic layer was dried over sodium sulphate, filtered, and the solvent was removed under vacuum to give the desired product (0.74 g). HPLC Rt: 7.05; LCMS (M+1): 595; Yield: 86%.

### Stage-8

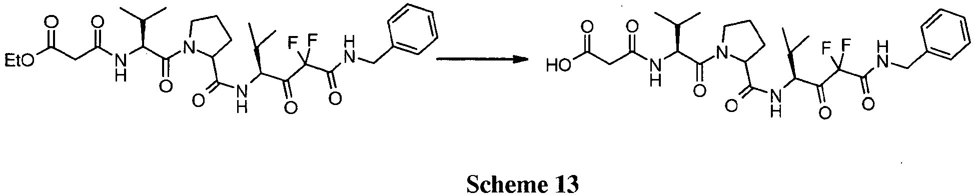

A solution of stage-7 (0.75 g, 1.2 mmol) in methanol water 1:1 (5 mL:5 mL) was treated with 1N NaOH (0.060 g, 1.5 mmol) and stirred at room temperature for 4 h. The reaction mixture was concentrated to remove methanol, acidified with 1N HCl (till pH 2) and extracted with ethyl acetate (50 mL). The organic layer was dried over sodium sulphate and concentrated under reduced pressure to give the desired product, which was then purified by preparative HPLC (0.4 g). HPLC Rt: 6.01-6.51; LCMS (M+1): 567; Yield: 56% After prep.

### Stage-9

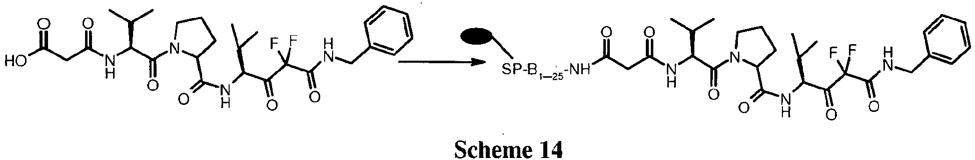

Wang resin (2 g, 0.08 mmol) was treated with 20% piperidine in DMF (20 mL) and stirred for about an hour. It was then washed with DMF (2 times), DCM (3 times) and then dried under reduced pressure. Kaiser test was performed on the resin to ensure complete removal of F-moc group. Then it was used for coupling with the stage -8. To a solution of the resin (2 g, 0.08 mmol) in DMF (5 mL) was added a solution of the above acid (0.1 g, 0.16 mmol) in DMF (3 mL), followed by the addition of PyBoP(0.83 g, 0.16 mmol) and N-methyl morpholine (0.2 mL, 0.2 mmol). The reaction mixture was allowed to shake on a shaker for about 3 h. The solution was decanted, fresh lots of the above reagents were again added and it was further allowed to shake for 3 h. This process was repeated for about 4 times. The solution was decanted, and the resin was washed with DMF (3 times), DCM (3 times) dried under reduced pressure. Kaiser test was performed on the resin to ensure that the coupling has taken place.

### Stage-10

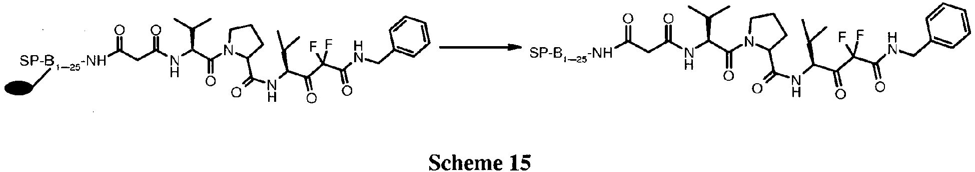

Procedure for making the cleavage solution. TFA (81%), Phenol (5%), Thioanisole (5%), 1,2,Ethanedithiol (2.5%), Water (3%), Dimethylsulphide (2%), ammonium iodide (1.5%). To the resin was added the cleavage solution (25 mL) and was allowed to shake on a shaker for about 3 h. The resin was filtered through cotton and washed with TFA. The filtrate was then concentrated under reduced pressure and triturated with cold ether to give a white solid, which was then purified by preparative HPLC to give the desired product (0.080 g). LCMS (M/3): 1160; Yield: 2.4%.

### Example 4: Bronchoaveolar Lavage of the Lungs following HNE Exposure

A total of 14 animals were used in this study. Anesthetized mice received 0.1 ml of solution delivered by a blunt tip catheter to the opening of the trachea and the animals allowed to aspirate the solution into their lungs. All animals were exposed to the treatment for 2 hours and re-anesthetized. Bronchoalveolar lavage of the lungs was performed as described below. The four animals given HNE (the causative agent of emphysema) had an average lung blood cell level by weight of 0.053 grams. Target 2 (a potent HNE inhibitor developed by Zeneca in the early 1990s) when given with the HNE, reduced the average blood level in the lungs by 34% to 0.035 grams. Target C (the Zeneca molecule covalently attached to the N-terminus of the first 25 residues of the human surfactant B peptide) when given with the HNE reduced the average blood level in the lungs by 87% to 0.007 grams.

A set of animals was used to determine the optimal concentration of HNE for the study. An initial sample exposed to 50 micrograms of HNE did not survive exposure. The amount of HNE was reduced to 40 micrograms to maintain a maximal detrimental but survivable effect. The animals exposed to 40 micrograms of HNE were able to survive long enough to perform the complete study. All animals remaining in the complete study were exposed to 40 micrograms of HNE.

Target 2 (the Zeneca molecule) was given in a 70-fold molar excess relative to HNE. In the kinetic studies, Target 2 has 2 nM affinity. Target C (the Zeneca molecule covalently attached to the first 25 residues of the human surfactant peptide B) loses approximately two orders of magnitude of potency as compared to Target 2 in the kinetic studies and thus Target C has approximately 200 nM affinity for HNE. Accordingly, the Target C was given to the animals in a 100 fold molar excess relative to HNE in these studies.

Bronchio-Alveolar lavage (BAL) is performed by 1) anesthetizing the animals; 2) performing a tracheotomy on the animals and cutting open the chest and spreading the ribs to enable full lung expansion; 3) pressurizing 1ml of PBS buffer solution into the lungs through the tracheotomy catheter; 4) sucking the solution out of the lungs; 5) centrifuging the recovered solution to separate the red blood cell fraction from the liquid fraction; and 6) weighing the red blood cell fraction.

**Table 2: Lung Blood Cell Level Measurements**

| **Treatment** | Mouse group 1 | Mouse group 2 | Mouse group 3 | Mouse Group 4 | Group mean |
|---|---|---|---|---|---|
| PBS/DMSO | 0 | 0 | 0 | Not done | 0 |
| HNE/DMSO | 0.09 g | 0.049 g | 0.021 g | 0.051 g | 0.053 g |
| Target 2+HNE | 0.03 g | 0.041 g | 0.03 g | 0.039 g | 0.035 g |
| Target C+HNE | 0 | 0.007 g | 0.014 g | Not done | 0.007 g |

### EQUIVALENTS

The invention may be embodied in other specific forms without departing from the spirit or essential characteristics thereof. The foregoing embodiments are therefore to be considered in all respects illustrative rather than limiting on the invention described herein. Scope of the invention is thus indicated by the appended claims rather than by the foregoing description, and all changes that come within the meaning and range of equivalency of the claims are intended to be embraced therein.

**Table 1: Exemplary human SP-B peptides (C-terminal truncations)**

| Human SP-B peptides | SEQ ID NO |
|---|---|
| FPIPLPYCWLCRALIKRIQAMIPKGALAVAVAQVCRVVPLVAGGICQCLAERYS VILLDTLLGRMLPQLVCRLVLRC | SEQ ID NO: 36 |
| FPIPLPYCWLCRALIKRIQAMIPKGALAVAVAQVCRVVPLVAGGICQCLAERYS VILLDTLLGRMLPQLVCRLVLR | SEQ ID NO: 37 |
| FPIPLPYCWLCRALIKRIQAMIPKGALAVAVAQVCRVVPLVAGGICQCLAERYS VILLDTLLGRMLPQLVCRLVL | SEQ ID NO: 38 |
| FPIPLPYCWLCRALIKRIQAMIPKGALAVAVAQVCRVVPLVAGGICQCLAERYS VILLDTLLGRMLPQLVCRLV | SEQ ID NO: 39 |
| FPIPLPYCWLCRALIKRIQAMIPKGALAVAVAQVCRVVPLVAGGICQCLAERYS VILLDTLLGRMLPQLVCRL | SEQ ID NO: 40 |
| FPIPLPYCWLCRALIKRIQAMIPKGALAVAVAQVCRVVPLVAGGICQCLAERYS VILLDTLLGRMLPQLVCR | SEQ ID NO: 41 |
| FPIPLPYCWLCRALIKRIQAMIPKGALAVAVAQVCRVVPLVAGGICQCLAERYS VILLDTLLGRMLPQLVC | SEQ ID NO: 42 |
| FPIPLPYCWLCRALIKRIQAMIPKGALAVAVAQVCRVVPLVAGGICQCLAERYS VILLDTLLGRMLPQLV | SEQ ID NO: 43 |
| FPIPLPYCWLCRALIKRIQAMIPKGALAVAVAQVCRVVPLVAGGICQCLAERYS VILLDTLLGRMLPQL | SEQ ID NO: 44 |
| FPIPLPYCWLCRALIKRIQAMIPKGALAVAVAQVCRVVPLVAGGICQCLAERYS VILLDTLLGRMLPQ | SEQ ID NO: 45 |
| FPIPLPYCWLCRALIKRIQAMIPKGALAVAVAQVCRVVPLVAGGICQCLAERYS VILLDTLLGRMLP | SEQ ID NO: 46 |
| FPIPLPYCWLCRALIKRIQAMIPKGALAVAVAQVCRVVPLVAGGICQCLAERYS VILLDTLLGRML | SEQ ID NO: 47 |
| FPIPLPYCWLCRALIKRIQAMIPKGALAVAVAQVCRVVPLVAGGICQCLAERYS VILLDTLLGRM | SEQ ID NO: 48 |
| FPIPLPYCWLCRALIKRIQAMIPKGALAVAVAQVCRVVPLVAGGICQCLAERYS VILLDTLLGR | SEQ ID NO: 49 |
| FPIPLPYCWLCRALIKRIQAMIPKGALAVAVAQVCRVVPLVAGGICQCLAERYS VILLDTLLG | SEQ ID NO: 50 |
| FPIPLPYCWLCRALIKRIQAMIPKGALAVAVAQVCRVVPLVAGGICQCLAERYS VILLDTLL | SEQ ID NO: 51 |
| FPIPLPYCWLCRALIKRIQAMIPKGALAVAVAQVCRVVPLVAGGICQCLAERYS VILLDTL | SEQ ID NO: 52 |
| FPIPLPYCWLCRALIKRIQAMIPKGALAVAVAQVCRVVPLVAGGICQCLAERYS VILLDT | SEQ ID NO: 53 |
| FPIPLPYCWLCRALIKRIQAMIPKGALAVAVAQVCRVVPLVAGGICQCLAERYS VILLD | SEQ ID NO: 54 |
| FPIPLPYCWLCRALIKRIQAMIPKGALAVAVAQVCRVVPLVAGGICQCLAERYS VILL | SEQ ID NO: 55 |
| FPIPLPYCWLCRALIKRIQAMIPKGALAVAVAQVCRVVPLVAGGICQCLAERYS VIL | SEQ ID NO: 56 |
| FPIPLPYCWLCRALIKRIQAMIPKGALAVAVAQVCRVVPLVAGGICQCLAERYS VI | SEQ ID NO: 57 |
| FPIPLPYCWLCRALIKRIQAMIPKGALAVAVAQVCRVVPLVAGGICQCLAERYS V | SEQ ID NO: 58 |
| FPIPLPYCWLCRALIKRIQAMIPKGALAVAVAQVCRVVPLVAGGICQCLAERYS | SEQ ID NO: 59 |
| FPIPLPYCWLCRALIKRIQAMIPKGALAVAVAQVCRVVPLVAGGICQCLAERY | SEQ ID NO: 60 |
| FPIPLPYCWLCRALIKRIQAMIPKGALAVAVAQVCRVVPLVAGGICQCLAER | SEQ ID NO: 61 |
| FPIPLPYCWLCRALIKRIQAMIPKGALAVAVAQVCRVVPLVAGGICQCLAE | SEQ ID NO: 62 |
| FPIPLPYCWLCRALIKRIQAMIPKGALAVAVAQVCRVVPLVAGGICQCLA | SEQ ID NO: 63 |
| FPIPLPYCWLCRALIKRIQAMIPKGALAVAVAQVCRVVPLVAGGICQCL | SEQ ID NO: 64 |
| FPIPLPYCWLCRALIKRIQAMIPKGALAVAVAQVCRVVPLVAGGICQC | SEQ ID NO: 65 |

| Human SP-B peptides | SEQ ID NO |
|---|---|
| FPIPLPYCWLCRALIKRIQAMIPKGALAVAVAQVCRVVPLVAGGICQ | SEQ ID NO: 66 |
| FPIPLPYCWLCRALIKRIQAMIPKGALAVAVAQVCRVVPLVAGGIC | SEQ ID NO: 67 |
| FPIPLPYCWLCRALIKRIQAMIPKGALAVAVAQVCRVVPLVAGGI | SEQ ID NO: 68 |
| FPIPLPYCWLCRALIKRIQAMIPKGALAVAVAQVCRVVPLVAGG | SEQ ID NO: 69 |
| FPIPLPYCWLCRALIKRIQAMIPKGALAVAVAQVCRVVPLVAG | SEQ ID NO: 70 |
| FPIPLPYCWLCRALIKRIQAMIPKGALAVAVAQVCRVVPLVA | SEQ ID NO: 71 |
| FPIPLPYCWLCRALIKRIQAMIPKGALAVAVAQVCRVVPLV | SEQ ID NO: 72 |
| FPIPLPYCWLCRALIKRIQAMIPKGALAVAVAQVCRVVPL | SEQ ID NO: 73 |
| FPIPLPYCWLCRALIKRIQAMIPKGALAVAVAQVCRVVP | SEQ ID NO: 74 |
| FPIPLPYCWLCRALIKRIQAMIPKGALAVAVAQVCRVV | SEQ ID NO: 75 |
| FPIPLPYCWLCRALIKRIQAMIPKGALAVAVAQVCRV | SEQ ID NO: 76 |
| FPIPLPYCWLCRALIKRIQAMIPKGALAVAVAQVCR | SEQ ID NO: 77 |
| FPIPLPYCWLCRALIKRIQAMIPKGALAVAVAQVC | SEQ ID NO: 78 |
| FPIPLPYCWLCRALIKRIQAMIPKGALAVAVAQV | SEQ ID NO: 79 |
| FPIPLPYCWLCRALIKRIQAMIPKGALAVAVAQ | SEQ ID NO: 80 |
| FPIPLPYCWLCRALIKRIQAMIPKGALAVAVA | SEQ ID NO: 81 |
| FPIPLPYCWLCRALIKRIQAMIPKGALAVAV | SEQ ID NO: 82 |
| FPIPLPYCWLCRALIKRIQAMIPKGALAVA | SEQ ID NO: 83 |
| FPIPLPYCWLCRALIKRIQAMIPKGALAV | SEQ ID NO: 84 |
| FPIPLPYCWLCRALIKRIQAMIPKGALA | SEQ ID NO: 85 |
| FPIPLPYCWLCRALIKRIQAMIPKGAL | SEQ ID NO: 86 |
| FPIPLPYCWLCRALIKRIQAMIPKGA | SEQ ID NO: 87 |
| FPIPLPYCWLCRALIKRIQAMIPKG | SEQ ID NO: 88 |
| FPIPLPYCWLCRALIKRIQAMIPK | SEQ ID NO: 89 |
| FPIPLPYCWLCRALIKRIQAMIP | SEQ ID NO: 90 |
| FPIPLPYCWLCRALIKRIQAMI | SEQ ID NO: 91 |
| FPIPLPYCWLCRALIKRIQAM | SEQ ID NO: 92 |
| FPIPLPYCWLCRALIKRIQA | SEQ ID NO: 93 |

## Claims

1. A drug composition formulated for inhalation, comprising:
a surface active agent **characterized by** an affinity for the human alveolar/gas interface, said surface active agent comprising at least a portion of a mammalian lung surfactant polypeptide or a mimic thereof substantially non-immunogenic to humans; and,
covalently bonded to said agent,
a pulmonary active drug which binds to an extracellular or cell surface-bound target accessible to the pulmonary/gas interface, wherein the pulmonary active drug is selected from: an elastase inhibitor, a corticosteroid, a bronchodilator, an antibiotic, and a chemotherapeutic agent.

2. The composition of claim 1, wherein the agent comprises a human lung surfactant polypeptide, a non human mammalian lung surfactant, for example a porcine or bovine lung surfactant polypeptide, a peptidomimetic comprising a deletion or amino acid substitution mutant of a mammalian lung surfactant polypeptide, or a fraction thereof.

3. The composition of claim 1, wherein the agent comprises a synthetic or recombinantly produced portion of the polypeptide component of a mammalian lung surfactant moiety.

4. The composition of claim 3, wherein the agent comprises at least a portion of SP-A, SP-B, SP-C, SP-D, or a mixture thereof, for example the 25 amino acid fragment from the N-terminus of SP-B.

5. The composition of any one of the preceding claims further comprising a lipid.

6. The composition of claim 1, wherein said agent comprises or is derived from a mammalian lung surfactant harvested from the lungs of a mammal.

7. The composition of any of claims 1-6, wherein the pulmonary active drug is a corticosteroid selected from: betamethasone, budesonide, cortisone, dexamethasone, hydrocortisone, methylprednisolone, prednisolone, prednisone, and triamcinolone.

8. The composition of any of claims 1-6, wherein the pulmonary active drug is a bronchodilator selected from: salbutamol, albuterol, terbutaline, fenoterol, fenoterol hydrobromide, rimiterol, reproterol, pirbuterol, isoprenaline, orciprenaline, bitolterol, broxaterol, salmeterol, salmeterol xinafoate, formoterol, formoterol fumarate, clenbuterol, procaterol, ipratropium, ipratropium bromide, tiotropium, tiotropium bromide monohydrate, aminophyliline, iorciprenaline, oxtriphylline, terbutaline sulfate, and theophylline.

9. The composition of any of claims 1-6 wherein the pulmonary active drug is an antibiotic selected from: amoxicillin, ampicillin, bacampicillin, carbenicillin, carbenicillin indanyl, mezlocilin, piperacillin, ticarcilin, antoxicillin-clavulanic acid, ampicillin-sulbactam, benzylpenicillin, cloxacilin, dicloxacilin, phenoxymethylpenicillin, carbenicillin, methicillin, oxacilin, penicilin G (benzathine, potassium, procaine), penicilin V, propicillin, epicillin, cyclacillin, piperacilin plus tazobactam, ticarcilllin plus clavulanic acid, naficillin, cefadroxil, cefazolin, cephalexin, cephalothin, cephapirin, cephradine, cefaclor, cefamandole, cefoicid, ceforanide, cefoxitin, cefprozil, ceftmetazole, cefuroxime, cefuoxime axetil, loracarbef, cefdinir, ceftibuten, cefditoren, cefatamet, cefoperazone, cefixime, cefotaxime, cefpodoxime, ceftazidime, ceftibuten, ceftizoxime, ceftriaxone, cefepime, azithromycin, clarithromycin, clindamycin, dirithromycin, erythromycin, lincomycin, telithromycine, troleandomycin, aztreonam, doripenem, imipenem-cilastatin, meropenem, amikacin, amikacin sulfate, gentamicin, genatmicin sulfate, kanamycin, metilmicin, neomycin, netilmicin, streptomycin, tobramycin, paromycin, dalbavancin, oritavancin, telavancin, teicoplanin, vancomycin, demclocylline, doxycycline, methacyline, minocyline, oxytetracycline, tetracyline, chloretracycline, linezolid, quinoprisitin plus dalfopristin, mafenide, silver sulfadiazine, sulfacetamide, sulfadiazine, sulfamethoxazole, sulfasalzine, sulfanilamide, sulfisoxazole, trimethoprim-sulfamethoxazole, sulfamethizole, bacitracin, chloramphenical, colistemetate, fosfomycin, isoniazid, methenamine, metronidazol, mupirocin, nitrofurantoin, nitrofurazone, novobiocin, polymyxin B, spectinomycin, trimethoprim, colistin, cycloserine, capreomycin, pyrazinamide, para-aminosalicyclic acid, erythromycin ethylsuccinate plus sulfisoxazole, and tigecycline.

10. The composition of any of claims 1-6 wherein the pulmonary active drug is a chemotherapeutic agent selected from: alkylating agents, antiestrogens, aclarubicin, actinomycin D, aldesleukin, aleantuzumab, alitretinoin, allopurinol, altretamine, amifostine, anastrozole, asparaginase, bexarotene, bisantrene, bleomycin, busulfan, BCNU (carmustine), calusterone, capecitabine, carboplatin, celecoxib, chlorambucil, cisplatin, cladribine, cyclophosphamide, cyclooxygenase-2 inhibitor, cytarabine, CCNU (lomustine), dacarbazine, daunorubine, daunomycin, denileukin diftitox, dexrazoxane, diaziquone, docetaxel, doxorubicin, epirubicin, epoetin alfa, esorubicin. estramustine, etoposide (VP-16), exemestane, Filgrastim, floxuridine, fludarabine, 5-fluorouracil, fulvestrant, galactitol, gemcitabine, gemtuzumab, goserelin acetate, hydroxyurea, ibritumomab tiuxetan, idarubicin, ifosfamide, imatinib mesylate, interferon alpha, interferon gamma, iriniotecan, iroplatin, letrozole, leucovorin, levamisole, lonidamine, megrestrol acetate, melphalan, mercaptopurine, mesna, methotrexate, methoxsalen, mitomycin C, mitotane, mitoxantrone, mitoguazone, nandrolone phenpropionate, Nofetumomab, nitrogen mustard, oprelvekin, oxaliplatin, paclitaxel, pamidronate, pegademase, pegaspargase, pegfilgrastim, pentostatin, pipobroman, plicamycin, porfimer sodium, procarbazine, progestins, prednimustine, PCNU, quinacrine, rasburicase, rituximab, sargramostim, streptozocin, talc, tamoxifen, temozolomide, teniposide (VM-26), testolactone, thioguanine, thiotepa, topotecan, toremifene, tositumomab, trastuzumab, tertinoin, uracil mustard, valrubicin, vinblastine, vincristine, vindesine, vinorelbine, and zoledronate.

11. The composition of any of the preceding claims disposed in an inhalation device for use by a human patient.

12. The composition of any one of claims 1-11 for use in the treatment by inhalation of a disease or condition of the human lung.

13. The composition of claim 12, wherein said composition is administered once daily, every other day, every three days, every five days, or weekly.

14. The composition of claim 12 or 13, wherein said administration reduces systemic bioavailability of the drug relative to inhalation administration of unconjugated drug.

15. The composition of any one of claims 12 to 14, wherein said administration increases residence time of the drug in the lung relative to inhalation administration of unconjugated drug.

## Patentansprüche

1. Arzneimittelzusammensetzung formuliert zum Inhalieren, umfassend:
einen oberflächenaktiven Stoff **gekennzeichnet durch** eine Affinität für die humane Alveolar-Gas-Grenzfläche, wobei der oberflächenaktive Stoff wenigstens einen Teil eines Säugetierlungen Netzmittelpolypeptids oder einem Imitat davon umfasst, das für Menschen im Wesentlichen nicht immunogen ist, und, an den Stoff kovalent gebunden,
ein lungenwirksames Arzneimittel, das an ein extrazelluläres oder an ein an einer Zelloberfläche gebundenes Ziel bindet, das **durch** die Alveolar-Gas-Grenzfläche erreichbar ist, wobei das lungenwirksame Arzneimittel ausgewählt ist unter einem Elastaseinhibitor, einem Corticosteroid, einem Bronchospasmolytikum, einem Antibiotikum und einem chemotherapeutischen Mittel.

2. Zusammensetzung nach Anspruch 1, wobei der Stoff ein menschliches Lungen Netzmittelpolypeptid, ein nichtmenschliches Säugetrierlungen Netzmittelpolypeptid beispielsweise ein Schweine- oder Rinderlungen Netzmittelpolypeptid, ein Peptidmimetikum umfassend eine Deletions- oder eine Aminosäurenaustausch-Mutante eines menschliches Lungen Netzmittelpolypeptids oder eine Fraktion davon umfasst.

3. Zusammensetzung nach Anspruch 1, wobei der Stoff einen künstlich oder rekombinant hergestellten Teil der Polypeptidkomponente eines Säugetierlungen Netzmittelrests umfasst.

4. Zusammensetzung nach Anspruch 3, wobei der Stoff wenigstens einen Teil von SP-A, SP-B, SP-C, SP-D oder ein Gemisch davon umfasst, beispielsweise das 25 Aminosäurenfragment des N-Terminus von SP-B.

5. Zusammensetzung nach einem der vorstehenden Ansprüche, ferner ein Lipid umfassend.

6. Zusammensetzung nach Anspruch 1, wobei der Stoff ein Säugetierlungen Netzmittel erhalten aus den Lungen eines Säugetiers umfasst oder davon angeleitet ist.

7. Zusammensetzung nach einem der Ansprüche 1-6, wobei das lungenwirksame Arzneimittel ein Corticosteroid ausgewählt unter Betamethason, Budesonid, Cortison, Dexamethason, Hydrocortison, Methylprednisolon, Prednisolon, Prednison und Triamcinolon ist.

8. Zusammensetzung nach einem der Ansprüche 1-6, wobei das lungenwirksame Arzneimittel ein Bronchospasmolytikum ausgewählt unter Salbutamol, Albuterol, Terbutalin, Fenoterol, Fenoterolhydrobromid, Rimiterol, Reproterol, Pirbuterol, Isoprenalin, Orciprenalin, Bitolterol, Broxaterol, Salmeterol, Salmeterolxinafoat, Formoterol, Formoterolfumarat, Clenbuterol, Procaterol, Ipratropium, Ipratropiumbromid, Tiotropium, Tiotropiumbromid-Monohydrat, Aminophylilin, Iorciprenalin, Oxtriphyllin, Terbutalinsulfat und Theophyllin ist.

9. Zusammensetzung nach einem der Ansprüche 1-6, wobei das lungenwirksame Arzneimittel ein Antibiotikum ausgewählt unter Amoxicillin, Ampicillin, Bacampicillin, Carbenicillin, Carbenicillin Indanyl, Mezlocilin, Piperacillin, Ticarcilin, Amoxicillin-Clavulansäure, Ampicillinsulbactam, Benzylpenicillin, Cloxacilin, Dicloxacilin, Phenoxymethylpenicillin, Carbenicillin, Methicillin, Oxacilin, Penicilin G (Benzathin, Kalium, Procain), Penicilin V, Propicillin, Epicillin, Cyclacillin, Piperacilin mit Tazobactam, Ticarcilllin mit Clavulansäure, Naficillin, Cefadroxil, Cefazolin, Cephalexin, Cephalothin, Cephapirin, Cephradin, Cefaclor, Cefamandol, Cefoicid, Ceforanide, Cefoxitin, Cefprozil, Ceftmetazol, Cefuroxim, Cefuoximaxetil, Loracarbef, Cefdinir, Ceftibuten, Cefditoren, Cefatamet, Cefoperazon, Cefixim, Cefotaxim, Cefpodoxim, Ceftazidim, Ceftibuten, Ceftizoxim, Ceftriaxon, Cefepim, Azithromycin, Clarithromycin, Clindamycin, Dirithromycin, Erythromycin, Lincomycin, Telithromycin, Troleandomycin, Aztreonam, Doripenem, Imipenem-Cilastatin, Meropenem, Amikacin, Amikacinsulfat, Gentamicin, Genatmicinsulfat, Kanamycin, Metilmicin, Neomycin, Netilmicin, Streptomycin, Tobramycin, Paromycin, Dalbavancin, Oritavancin, Telavancin, Teicoplanin, Vancomycin, Demclocyllin, Doxycyclin, Methacylin, Minocylin, Oxytetracyclin, Tetracyclin, Chloretracyclin, Linezolid, Quinoprisitin mit Dalfopristin, Mafenid, Silbersulfadiazin, Sulfacetamid, Sulfadiazin, Sulfamethoxazol, Sulfasalzin, Sulfanilamid, Sulfisoxazol, Trimethoprim-Sulfamethoxazol, Sulfamethizol, Bacitracin, Chloramphenicol, Colistemetat, Fosfomycin, Isoniazid, Methenamin, Metronidazol, Mupirocin, Nitrofurantoin, Nitrofurazone, Novobiocin, Polymyxin B, Spectinomycin, Trimethoprim, Colistin, Cycloserin, Capreomycin, Pyrazinamid, Paraaminosalicylsäure, Erythromycinethylsuccinat mit Sulfisoxazol und Tigecyclin ist.

10. Zusammensetzung nach einem der Ansprüche 1-6, wobei das lungenwirksame Arzneimittel ein Chemotherapeutikum ausgewählt unter Alkylierungsmitteln, Antiöstrogenen, Aclarubicin, Actinomycin D, Aldesleukin, Alemtuzumab, Alitretinoin, Allopurinol, Altretamin, Amifostin, Anastrozol, Asparaginase, Bexaroten, Bisantren, Bleomycin, Busulfan, BCNU (Carmustin), Calusteron, Capecitabin, Carboplatin, Celecoxib, Chlorambucil, Cisplatin, Cladribin, Cyclophosphamid, Cyclooxygenase-2 Inhibitor, Cytarabine, CCNU (Lomustin), Dacarbazin, Daunorubin, Daunomycin, Denileukin-Diftitox, Dexrazoxan, Diaziquon, Docetaxel, Doxorubicin, Epirubicin, Epoetin alpha, Esorubicin, Estramustin, Etoposid (VP-16), Exemestan, Filgrastim, Floxuridin, Fludarabin, 5- Fluorouracil, Fulvestrant, Galactitol, Gemcitabin, Gemtuzumab, Goserelinazetat, Hydroxyurea, Ibritumomabtiuxetan, Idarubicin, Ifosfamid, Imatinibmesylat, Interferon alpha, Interferon gamma, Iriniotecan, Iroplatin, Letrozole, Leucovorin, Levamisole, Lonidamin, Megrestrolazetat, Melphalan, Mercaptopurin, Mesna, Methotrexat, Methoxsalen, Mitomycin C, Mitotan, Mitoxantron, Mitoguazon, Nandrolonphenpropionat, Nofetumomab, Stickstoff-Lost, Oprelvekin, Oxaliplatin, Paclitaxel, Pamidronat, Pegademase, Pegaspargase, Pegfilgrastim, Pentostatin, Pipobroman, Plicamycin, Porfimernatrium, Procarbazin, Progestine, Prednimustin, PCNU, Quinacrin, Rasburicase, Rituximab, Sargramostim, Streptozocin, Talk, Tamoxifen, Temozolomid, Teniposid (VM-26), Testolacton, Thioguanin, Thiotepa, Topotecan, Toremifen, Tositumomab, Trastuzumab, Tertinoin, Uracil-Senf, Valrubicin, Vinblastin, Vincristin, Vindesin, Vinorelbin und Zoledronat ist.

11. Zusammensetzung nach einem der vorstehenden Ansprüche, angeordnet in einer Inhalationsvorrichtung zur Verwendung durch einen menschlichen Patienten.

12. Zusammensetzung nach einem der Ansprüche 1-11 zur Verwendung in der Behandlung einer Erkrankung oder eines Zustandes der menschlichen Lunge mittels Inhalation.

13. Zusammensetzung nach Anspruch 12, wobei die Zusammensetzung einmal täglich, jeden zweiten Tag, jeden dritten Tag, jeden fünften Tag oder wöchentlich verabreicht wird.

14. Zusammensetzung nach Anspruch 12 oder 13, wobei die Verabreichung die systemische Bioverfügbarkeit des Arzneimittels relativ zu einer Verabreichung durch Inhalation eines nicht konjugierten Arzneimittels verringert.

15. Zusammensetzung nach Anspruch 12 bis 14, wobei die Verabreichung die Verweildauer des Arzneimittels in der Lunge relativ zu einer Verabreichung durch Inhalation eines nicht konjugierten Arzneimittels erhöht.

## Revendications

1. Composition de médicaments formulée pour une inhalation, comprenant :
un agent tensioactif **caractérisé par** une affinité pour l'interface alvéolaire/gazeuse humaine, ledit agent tensioactif comprenant au moins une partie d'un polypeptide tensioactif pulmonaire mammifère ou un imitateur de celui-ci essentiellement non immunogène pour les êtres humains ; et,
lié par covalence audit agent,
un médicament pulmonaire actif qui se lie à une cible liée à une surface extracellulaire ou cellulaire accessible à l'interface pulmonaire/gazeuse, dans laquelle le médicament pulmonaire actif est choisi parmi : un inhibiteur d'élastase, un corticostéroïde, un bronchodilatateur, un antibiotique et un agent chimiothérapeutique.

2. Composition selon la revendication 1, dans laquelle l'agent comprend un polypeptide tensioactif pulmonaire humain, un tensioactif pulmonaire mammifère non humain, par exemple un polypeptide tensioactif pulmonaire porcin ou bovin, un composé peptidomimétique comprenant une mutation par délétion ou substitution d'acides aminés d'un polypeptide tensioactif pulmonaire mammifère, ou une fraction de celui-ci.

3. Composition selon la revendication 1, dans laquelle l'agent comprend une partie produite par voie synthétique ou par recombinaison du composant polypeptidique d'un groupement tensioactif pulmonaire mammifère.

4. Composition selon la revendication 3, dans laquelle l'agent comprend au moins une partie de SP-A, de SP-B, de SP-C, de SP-D ou un mélange de ceux-ci, par exemple le fragment à 25 acides aminés à partir de l'extrémité N de SP-B.

5. Composition selon l'une quelconque des revendications précédentes, comprenant en outre un lipide.

6. Composition selon la revendication 1, dans laquelle ledit agent comprend ou est dérivé d'un tensioactif pulmonaire mammifère récolté dans les poumons d'un mammifère.

7. Composition selon l'une quelconque des revendications 1 à 6, dans laquelle le médicament pulmonaire actif est un corticostéroïde choisi parmi : la bétaméthasone, le budésonide, la cortisone, la dexaméthasone, l'hydrocortisone, la méthylprednisone, la prednisolone, la prednisone et la triamcinolone.

8. Composition selon l'une quelconque des revendications 1 à 6, dans laquelle le médicament pulmonaire actif est un bronchodilatateur choisi parmi : le salbutamol, l'albutérol, la terbutaline, le fénotérol, le bromhydrate de fénotérol, le rimitérol, le réprotérol, le pirbutérol, l'isoprénaline, l'orciprénaline, le bitoltérol, le broxatérol, le salmétérol, le xinafoate de salmétérol, le formotérol, le fumarate de formotérol, le clenbutérol, le procatérol, l'ipratropium, le bromure d'ipratropium, le tiotropium, le bromure de tiotropium monohydraté, l'aminophyliline, l'iorciprénaline, l'oxtriphylline, le sulfate de terbutaline et la théophylline.

9. Composition selon l'une quelconque des revendications 1 à 6, dans laquelle le médicament pulmonaire actif est un antibiotique choisi parmi : l'amoxicilline, l'ampicilline, la bacampicilline, la carbénicilline, le carbénicilline indanyle, la méziociline, la pipéracilline, la ticarciline, l'amoxicilline-acide clavulanique, l'ampicilline-sulbactame, la benzylpénicilline, la cloxaciline, la dicloxaciline, la phénoxyméthylpénucilline, la carbénicilline, la méthicilline, l'oxacilline, la péniciline G (benzathine, potassium, procaïne), la péniciline V, la propicilline, l'épicilline, la cyclacilline, la pipéraciline plus le tazobactame, la ticarcilline plus l'acide clavulanique, la naficilline, le cédadroxil, la céfazoline, la céphalexine, la céphalothine, la céphapirine, la céphradine, le céfactor, le céfamandole, le céfoicid, le céforanide, la céfoxitine, le célprozil, le céftmétazole, la féfuroxime, le céfuroxime axétif, le loracarbef, le cefdinir, le ceftibuten, le cefditoren, le céfatamet, la céfopérazone, le céfixime, le céfotaxime, le cefpodoxime, le céftazidime, le ceftibuten, le ceftizoxime, la ceftriaxone, le céfépime, l'azithromycine, la clarithromycine, la clindamycine, la dirithromycine, l'éryhtromycine, la lincomycine, la télithromycine, la troléandomycine, l'azrtéonam, le doripénem, l'imipénem-cilastatine, le méropénem, l'amikacine, le sulfate d'amikacine, la gentamicine, le sulfate de gentamicine, la kanamycine, la métilmicine, la néomycine, la nétilmicine, la treptomycine, la tobramycine, la paromycine, la dalbavancine, l'oritavancine, la talavancine, la téicoplanine, la vamcomycine, la démélocylline, la doxycycline, la méthacyline, la minocyline, l'oxytétracycline, la tétracycline, la chlorétracycline, le linézolid, la quinaprisitine plus la dalfopristine, le mafénide, la sulfadiazine d'argent, le sulfacétamide, la sulfadiazine, le sulfaméthoxazole, la sulfasalzine, la sulfanilamide, le sulfisoxazole, le triméthoprim-sulfaméthoxazole, le sulfaméthizole, la bacitracine, le chloramphénical, le colistémétate, la fosfomycine, l'isoniazid, la méthénamine, le métronidazol, la mupirocine, la nitrofurantoïne, la nitrofurazone, la novobiocine, la polymyxine B, la spectinomycine, le triméthoprim, la colistine, la cyclosérine, la capréomycine, le pyrazinamide, l'acide para-aminosalicylique, l'éthylsuccinate d'érythromycine plus le sulfisoxazole et la tigécycline.

10. Composition selon l'une quelconque des revendications 1 à 6, dans laquelle le médicament pulmonaire actif est un agent chimiothérapeutique choisi parmi : les agents alkylants, les antioestrogènes, l'aclarubicine, l'actinomycine D, l'aldésleukine, l'alemtuzumab, l'alitrétinoïne, l'allopurinol, l'altrétamine, l'aminofostine, l'anastrozole, l'asparaginase, le bexarotène, le bisantrène, la bléomycine, le busulfan, la BCNU (carmustine), la calustérone, la capécitabine, la carboplatine, le célécoxib, le chlorambucil, la cisplatine, la cladribine, le cyclophosphamide, l'inhibiteur de la cyclo-oxygénase-2, la cytarabine, le CCNU (lomustine), la dacarbazine, la daunorubine, la duanomycine, le dénileukine diftitox, le dexrazoxane, la diaziquone, le docétaxel, la doxorubine, l'épirubicine, l'époétine alpha, l'ésorubicine, l'estramustine, l'étoposide (VP-16), l'exémestane, la Fligrastime, la floxuridine, la fludarabine, le 5-fluorouracil la fulvestrant, le galactitol, la gemcitabine, le gemtuzumab, l'acétate de goséréline, l'hydroxy-urée, l'ibritumomab tiuxétan, l'idarubicine, l'ifosfamide, le mésylate d'imatinib, l'interféron alpha, l'interféron gamma, l'iriniotécan, l'iroplatine, le létrozole, la leucovorine, le lévamisole, la lonidamine, l'acétate de mégrestrol, le malphalan, la mercaptopurine, le mesna, la méthotréxate, la méthoxsalen, la mitomycine C, le mitotane, la mitoxantrone, la mitoguazone, le phénpropionate de nandrolone, le Nofétumomab, la moutarde azotée, l'oprelvékine, l'oxaliplatine, le paclitaxel, le pamidronate, la pégadémase, la pégaspargase, le pegfilgrastime, la pentostatine, le pipobroman, la plicamycine, le porfimer sodique, la procarbazine, les progestines, le prednimustine, le PCNU, la quinacrine, la rasburicase, le rituximab, la sargramostime, la streptozocine, le talc, le tamoxifen, le témozolomide, le ténoposide (VM-26), la testolactone, la thioguanine, la thiotépa, le topotécan, la torémifène, le tositumomab, le trastuzumab, la tertinoïne, la moutarde uracil, la viarubicine, la vinblastine, la vincristine, la vindésine, la vinorelbine et le zolédromate.

11. Composition selon l'une quelconque des revendications précédentes, disposée dans un dispositif d'inhalation destinée à une utilisation par un patient humain.

12. Composition selon l'une quelconque des revendications 1 à 11, destinée à une utilisation dans le traitement par inhalation d'une maladie ou d'une pathologie des poumons humains.

13. Composition selon la revendication 12, dans laquelle ladite composition est administrée une fois par jour, un jour sur deux, un jour sur trois, un jour sur cinq ou une fois par semaine.

14. Composition selon la revendication 12 ou 13, dans laquelle ladite administration réduit la biodisponibilité systémique du médicament par rapport à une administration par inhalation d'un médicament non conjugué.

15. Composition selon l'une quelconque des revendications 12 à 14, dans laquelle ladite administration augmente le temps de séjour du médicament dans les poumons par rapport à une administration par inhalation d'un médicament non conjugué.
